(19) Europäisches Patentamt
European Patent Office
Office européen des brevets

(11) EP 1 241 262 A2

(12) **EUROPEAN PATENT APPLICATION**

(43) Date of publication:
**18.09.2002 Bulletin 2002/38**

(51) Int Cl.[7]: **C12N 15/52**, C12N 15/82, A01H 5/00, A01H 5/10

(21) Application number: **02005639.6**

(22) Date of filing: **12.03.2002**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **13.03.2001 JP 2001070691**
**28.09.2001 JP 2001300038**

(71) Applicant: **Nara Institute of Science and Technology**
**Ikoma City, Nara Pref. (JP)**

(72) Inventors:
- **Sasaki, Yukiko A301, Mitsuke-momiji-en**
  **Nagoya City, Aichi Pref. (JP)**
- **Yokota, Akiho**
  **Ikoma City, Nara Pref. (JP)**
- **Madoka, Yuka 302, Sun Beam Fujinari**
  **Nagoya City, Aichi Pref. (JP)**

(74) Representative: **Klunker . Schmitt-Nilson . Hirsch**
**Winzererstrasse 106**
**80797 München (DE)**

Remarks:
The sequence listing, which is published as annex to the application documents, was filed after the date of filing. The applicant has declared that it does not include matter which goes beyond the content of the application as filed.

# (54) A method for promoting fatty acid synthesis in a plant

(57) According to the present invention, a novel method for promoting fatty acid content in a plant is provided. In this method, the promoter of *accD* gene of acetyl-CoA carboxylase was replaced with a promoter directing abundant expression in plastids and chloroplasts by using plastid transformation. This method increases the carboxyltransferase beta subunit protein encoded by the *accD* gene. Accompanied with it, the other subunits constituting the acetyl-CoA carboxylase apparently increases and this enzyme increases. Since the acetyl-CoA carboxylase is a key enzyme for rate-limiting step of fatty acid synthesis, synthesis of fatty acid can be promoted by the method of the present invention. The transformed plant produced according to the method of the present invention exhibits remarkable enhancement in the fatty acid content in leaves and seeds. Moreover, the leaf longevity extended and the number of the seeds per a plant body increased, thereby seed oil and productivity of the plant are improved.

*FIG. 5*

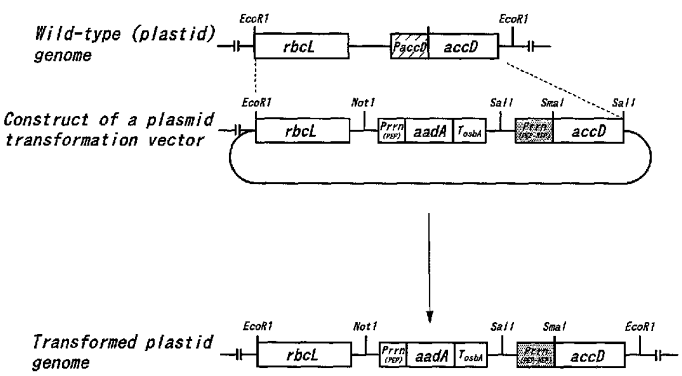


EP 1 241 262 A2

## Description

BACKGROUND OF THE INVENTION

1. Field of the invention

This invention relates to a method for promoting fatty acid content in a plant by over-expressing plastid-located *accD* gene encoding one subunit of plastidic acetyl-CoA carboxylase. Moreover, this invention relates to a transformed plant wherein fatty acid content increases using the method described above.

2. Prior art

The seed oils, different from petroleum, do not contribute to air pollution when subjected to combustion. Namely, photosynthesis products release the same amount of carbon dioxide fixed by assimilation and the carbon dioxide is circulated. Oils are energy-rich and not bulky compounds with hydrophobic property, compared with the other photosynthetic products such as starch and cellulose which contain crystal water. In this point, seed oils are the useful bio-mass and one of the candidates producing a clean energy. Seed oils are used not only for food resources but also for a variety of industrial materials, and it is important to increase seed oil contents. Seed oils are triglycerides composed of glycerol and fatty acids. The fatty acid composition in seeds is successfully altered by gene manipulation, and several transgenic plants have been developed to date.
In plants, carbon dioxide fixed by photosynthesis is stored as starch and oil. The products mainly stored in seeds are used for an energy resource for the next generation. There are two types of seeds, oil-rich seeds and the starch-rich seeds. The mechanism involved in the partitioning of the products into oil and starch has not been elucidated yet. However, it is considered that the amount of oil produced is, in part, dependent on the amount of acetyl-CoA carboxylase. Here, acetyl-CoA carboxylase is a key enzyme that participates in the initial rate-limiting step of fatty acid synthesis. Here, fatty acid is a main component of oil. The reaction catalyzed by acetyl-CoA carboxylase is as follows.

$$CH_3COSCoA \text{ (Acetyl-CoA)} + CO_2 \text{ --> } HOOCCH_2COSCoA \text{ (Malonyl-CoA)}$$

Malonyl-CoA, formed by this reaction, is mainly used for fatty acid synthesis. Palmitic acid of 16 carbons is formed by fatty acid synthase, from one molecule of acetyl-CoA and seven molecules of malonyl-CoA. In this biosynthesis, carbon chain elongation occurs sequentially with addition of two carbons, with generation of carbon dioxide at each step. The rate-limiting step of fatty acid synthesis is the step of malonyl-CoA formation and the activity of acetyl-CoA carboxylase is strictly controlled by this step in various organisms. The essential components of cells, such as glycerolipids of the membrane, triglycerides stored in seeds, cuticle lipids that prevent diffusion of water from epidermal cells, can be synthesized from fatty acids. The acetyl-CoA carboxylase is an important enzyme which supplies malonyl-CoA essential for cells. Thus the enzyme attracted attention of many researchers and attempts have been performed to increase the amount of the acetyl-CoA carboxylase in the purpose to activate fatty acid synthesis, which results in massive production of oils.
It has been known that in plants there are two types of acetyl-CoA carboxylase, namely eukaryotic form of acetyl-CoA carboxylase and prokaryotic form of acetyl-CoA carboxylase (referred to plastidic acetyl-CoA carboxylase). Structures of both enzymes are shown in Fig. 1. The present inventors have firstly identified the plastidic acetyl-CoA carboxylase in a plant at 1993 (J. Biol. Chem. 268, 25118-25123, 1993). The plastidic acetyl-CoA carboxylase consists of three dissociable components, i.e., biotin carboxylase (BC), biotin carboxyl carrier protein (BCCP) and carboxyltransferase (CT) alpha and beta subunits. Moreover, four genes encoding these components are designated as *accC, accB, accA* and *accD,* respectively. The plastidic acetyl-CoA carboxylase is a multi-enzyme complex with the molecular weight of about 500,000, composed of dimers of respective four subunits in case of pea. To the contrary, in the eukaryotic form of acetyl-CoA carboxylase, the requisite three activities are borne by single multi-functional polypeptide with the molecular weight of about 240,000. Moreover, the eukaryotic form of acetyl-CoA carboxylase consists of dimers of the polypeptide.
Incidentally, it has been known that the eukaryotic form enzyme is localized in cytosol and the plastidic acetyl-CoA carboxylase is localized in plastids. To increase acetyl-CoA carboxylase in plastids, the eukaryotic form of enzyme that designed to target into plastids was over-expressed by nuclear-transformation, which resulted in 5% increase in the oil content of the rapeseeds (Plant Physiol; 113, 75-81, 1997). This finding showed that the oil content might be increased by increasing the amount of acetyl-CoA carboxylase in plastids.
However, there was no successful gene manipulation of prokaryotic form of the enzyme to increase oil content. For efficient increase in the fatty acid synthesis, a technique that enables increase the amount of the plastidic acetyl CoA has been desired. To solve this problem, an object of the present invention is to provide a method available to increase the amount of plastidic acetyl- CoA carboxylase efficiently, via the technique of plastid transformation.

In tobacco, *accD, psaI, ycf4, cemA* and *petA* form a gene cluster and these genes are polycistronically transcribed by using the *accD* promoter. In the cluster, *psaI* encodes one of the proteins of Photosystem *I, ycf4* encodes a protein for Photosystem I accumulation, and *petA* encodes the chromosome f protein, but the biological function of *cemA* (a gene encoding chroloplast envelope membrane protein) is not yet understood. Replacement of the *accD* promoter presented here enhanced the expression of all these genes. Although the over-expression of *psaI, ycf4, cemA* and *petA* might affected the observed effects, the promoter engineering presented here offers a new method for not only the improvement of oil production but also an increase of plant yield.

## SUMMARY OF THE INVENTION

The first aspect of this invention relates to a method for promoting fatty acid synthesis in a plant, the method comprising over-expression of the *accD* gene of plastidic acetyl-CoA carboxylase. A further aspect of the present invention relates to the above-mentioned method comprising the steps of preparing a construct, the construct comprising a plasmid harboring (1) *accD* gene, (2) a promoter sequence of a gene exhibiting abundant expression in plastids and chloroplasts, and (3) a homologous sequence that enables homologous recombination in plastid genome and conducting plastid transformation of a plant by the construct to over-express the *accD* gene.

Another aspect of the present invention relates to the above-mentioned method wherein wherein over-expression of the *accD* gene causes increased carboxytransferase (CT) beta subunit accompanied with increased other subunits constituting plastidic acetyl-CoA carboxylase. Further aspect of this invention relates to the above-mentioned method wherein the other subunits constituting plastidic acetyl-CoA carboxylase are biotin carboxylase (BC) protein, biotin carboxyl-carrier protein (BCCP) and carboxytransferase (CT) alpha subunit protein.

Another aspect of the present invention relates to a transformed plant wherein chloroplasts of the plant are transformd by a construct comprising a plasmid harboring (1) *accD* gene, (2) a promoter sequence of a gene exhibiting abundant expression in plastids and chloroplasts, and (3) a homologous sequence that enables homologous recombination in plastid genome. Further aspect of this invention relates to the above mentioned transformed plant wherein the fatty acid content increases and the starch content decreases in leaves of the plant as compared with a plant of control line, and to the above mentioned transformed plant wherein the leaf longevity of the plant extended as compared with that of a plant of control line.

Moreover further aspect of this invention relates to the above mentioned transformed plant wherein the fatty acid content increases and the starch content decreases in seeds of the plant as compared with a plant of control line. Moreover, further aspect of this invention relates to the above mentioned transformed plant wherein the number of seeds per a plant body increases as compared with a plant of control line, thereby the amount of total fatty acids per the plant body increases as compared with a plant of control line, and to the above mentioned transformed plant wherein the ratio of fatty acids to starch is improved as compared with a plant of control line.

These and other objects and advantages of the invention will become more apparent upon a reading of the detailed description and drawings.

## BRIEF DESCRIPTION OF THE DRAWINGS

Fig. 1 is a schematic drawing showing structures of plastidic acetyl-CoA carboxylase and eukaryotic type acetyl-CoA carboxylase.

Fig. 2 is a figure showing the sequence of coding region of *accD* gene and its upstream region.

Fig. 3 is a figure showing the sequence of coding region of *rbcL* gene and its downstream region.

Fig. 4 is a figure showing the sequence of rrn 16 promoter.

Fig. 5 is a schematic drawing showing the structure of the construct for alteration of the accD promoter to be used for production of the line with *accD* abundant expression.

Fig. 6 is a schematic drawing showing the structure of the control construct to be used for production of the control line.

Fig. 7 is a photograph of genomic southern blot analysis showing introduction of the *accD* gene.

Fig. 8 is a photograph of genomic southern blot analysis showing introduction of the *aadA* gene.

Fig. 9 is a photograph of northern blot analysis showing expression of the *accD* gene.

Fig. 10 is a photograph of western blot analysis showing expression of each subunit of acetyl-CoA carboxylase.

Fig. 11 is a photograph of the 12-weeks old plants showing phenotype of the transformed plants.

Fig. 12 is a photograph of electronic microscope showing the distribution of starch granules and oil droplets in the chloroplast of the wild-type line.

Fig. 13 is a photograph of electronic microscope showing the distribution of starch granules and oil droplets in the chloroplast of the line with accD abundant expression.

Fig. 14 is a graph showing the results of fatty acid content analyzed on the leaves.

Fig. 15 is a graph showing the results of starch content analyzed on the leaves.

Fig. 16 is a graph showing the result of fatty acid content analyzed on the seeds.

Fig. 17 is a graph showing the result of starch content analyzed on the seeds.

Fig. 18 is a graph showing the fatty acid content per a plant (whole seeds).

Fig. 19 is a graph showing the starch content per a plant (whole seeds).

Fig. 20 is a graph showing the partitioning of fatty acid and starch, indicated by the ratio of total fatty acid content per total starch content.

## DETAILED DESCRIPTION OF THE INVENTION

The present inventors noticed the knowledge that the plastidic acetyl-CoA carboxylase (prokaryotic form) is composed of four subunits and only one subunit (*accD* subunit) is encoded by the plastid genome and the other subunits are encoded by nuclear genome, as mentioned above. To increase the acetyl-CoA carboxylase, expression of the four genes should be increased. However, it was shown that over-expression of the nuclear-encoded subunits did not increase the acetyl-CoA carboxylase level. This result suggests that the expression of the plastid-encoded subunit is limited and the amount of the enzyme is restricted. Therefore, the present inventors proposed that fatty acid synthesis would be promoted by increasing the expression of the *accD* gene and demonstrated the proposition.

That is, as shown in examples mentioned below, the original promoter of tobacco *accD* gene was removed and the promoter of ribosomal RNA (rRNA), exhibiting abundant expression in both plastids and chloroplasts, was ligated thereto. Then the resultant DNA was introduced into tobacco chloroplasts. The plastid transformation method developed by Maliga (Maliga et al., Proc. Natl. Acad. Sci. USA (1993) 90, 913-917) has been improved and practically applicable to tobacco plant. In general, the amount of expression of the *accD* gene is low, but it has been shown that the expression of *accD* gene can be increased using above-mentioned rRNA promoter.

To increase the amount of acetyl-CoA carboxylase, it is necessary to increase not only the amount of carboxyltransferase (CT) beta subunit protein encoded by *accD* gene, but also the amounts of other three subunits, namely biotin carboxylase (BC), biotin carboxyl carrier protein (BCCP) and carboxytransferrase (CT) alpha subunit, should be increased.

The genes encoding these three subunits are located in nuclear genome as mentioned above. According to the result of this investigation, accD subunit as well as these three subunits increased in the resultant transformants, indicating an increase in the acetyl-CoA carboxylase. Moreover, the oil content per seed also increased, whereby a novel method that enables alteration of the amount of oil was established. In the future, if massive production of seed oils will be achieved by molecular breeding, seed oils will partly take the place of the petroleum resource, whereby it will bear great contribution to the society. The reason that the nuclear-encoded subunits increased is considered to be as follows. In the wild type, three nuclear-encoded proteins are synthesized molar excess of the accD subunit, entered into plastids, and assembled with the accD subunit to form acetyl-CoA carboxylase. The excess proteins not assembled into the enzyme are decomposed immediately in the plastids. In the transformants obtained in this invention, the increased accD subunit can associate with the above-mentioned excess subunits to form the enzyme. Because the subunits that have been decomposed so far can form the stable enzyme, apparent increase of three subunits was observed. That is, the apparent increase in the other subunits is due to decrease in decomposition in the transformants.

In the method of the present invention, a construct is prepared. In the construct, three kinds of DNAs, namely the accD gene of the plastidic acetyl CoA carboxylase, a promoter sequence of a gene exhibiting abundant expression in both plastids and chloroplasts, and a homologous sequence that enables homologous recombination in plastid genome, are introduced into a plasmid. Here, the promoter sequence of a gene exhibiting abundant expression in plastids and chloroplasts is inserted into upstream of *accD* gene after removing the *accD* promoter, whereby it promotes expression of the *accD* gene. Incidentally, in the following examples, a chimeric spectinomycin resistant gene (a chimerid *aadA*: aminoglycoside 3"-adenylyl transferase gene: Svab, Z. & Maliga, P. (1993) Proc. Natl. Acad. Sci. USA 90, 913-917), which is a drug resistant gene, is also inserted into plasmid for selection of the transformants. In the present invention, it is preferred that a gene useful for the selection of the transformants be also inserted into the construct in such a manner. Then chloroplasts of a plant are transformed by the construct prepared as mentioned above. Preparation of the construct, whereon the objective gene is introduced, can be achieved using some suitable plasmid and some restriction enzymes, according to the technique usually used in this field of the art. The plasmid herein used is not to be specifically limited, and a plasmid which is generally used in this art, such as pZErO2.1, pUC18, pBluescript, can be utilized.

In the method of the present invention, a construct is prepared. In the construct, three kinds of DNAs, namely the *accD* gene of the plastidic acetyl CoA carboxylase, a promoter sequence of a gene exhibiting abundant expression in both plastids and chloroplasts, and a homologous sequence that enables homologous recombination in plastid genome, are introduced into a plasmid. Here, the promoter sequence of a gene exhibiting abundant expression in plastids and chloroplasts is inserted into upstream of *accD* gene after removing the accD promoter, whereby it promotes expression of the *accD* gene. Incidentally, in the following examples, a chimeric spectinomycin resistant gene (a chimeric *aadA*: aminoglycoside 3"-adenylyl transferase gene: Svab, Z. & Maliga, P. (1993) Proc. Natl. Acad. Sci. USA 90, 913-917), which is a drug resistant gene, is also inserted into plasmid for selection of the transformants. In the present invention, it is preferred that a gene useful for the selection of the transformants be also inserted into the construct in such a manner. Then plastids of a plant are transformed by the construct prepared as mentioned above. Preparation of the construct, whereon the objective gene is introduced, can be achieved using some suitable plasmid and some restriction enzymes, according to the technique usually used in this field of the art. The plasmid herein used is not to be specifically limited, and a plasmid which is generally used in this art, such as pZErO2.1, pUC18, pBluescript, can be utilized.

By the way, conventional plant transformation is performed by Agrobacterium-mediated transformation using cauliflower mosaic virus 35S promoter, which inserts the candidate gene into nuclear genome. In this transformation, constitutively expressed promoters, such as cauliflower mosaic virus 35S promoter, nopaline synthase terminator, are utilized, and the concern of so-called "penalty" remains. Therefore, a technique for molecular breeding utilizing alternative method without such concern has been required.

On the other hand, to over-express the plastid-located *accD* gene, the technique of plastid transformation is utilized in the present invention, which is one of the characteristic features of the present invention. The method used for the present invention is described in the literature of Maliga et al. (Maliga et al., Proc. Natl., Acad. Sci. USA (1993), 90, 913-917). In the examples, spectinomycin resistant gene has been also inserted for the selection described above, however, the concern of the "penalty" is assumed to be not problematic.

The plastid transformation used in the present invention is achieved by homologous recombination. Therefore, the gene can be inserted into a desired position of the genome, thus the characters of the resulting recombinants are assumed to be uniform. Incidentally, the term "homologous recombination" means recombination, which occurs between base sequences having substantially or completely the same sequences. In the conventional method of Agrobacterium-mediated transformation, the position of the introduced gene can not be expected and it is the defect of this method. As to this matter, the gene can be introduced into a specified site by using homologous sequence. Then the resulting transformants have a uniform character and there is no concern of causing troubles.

As mentioned above, to perform homologous recombination, it is necessary to introduce a certain sequence required for the recombination, simultaneously. In the following examples, a sequence comprising Rubisco large subunit (rbcL) gene and its downstream sequence adjacent to the upstream of the *accD* gene is used as the homologous sequence. For the *rbcL* gene exists at the upstream of the *accD* gene, homologous recombination can be successfully achieved using a sequence comprising the *accD* gene and the *rbcL* gene.

Homologous recombination is performed by a construct having a structure described below. That is, in the construct of the present invention, one homologous sequence present in the upstream of the objective sequence to be introduced and another homologous sequence present in its downstream are required. In the example mentioned below, one is the *rbcL* gene containing its downstream and another is the *accD* gene are the homologous sequence, and ribosomal RNA operon 16 promoter is the objective sequence to be introduced. Accordingly, the homologous recombination is not limited only to the *rbcL* gene and its downstream sequence and other sequences can be also utilized, so long as they can be utilized in the homologous recombination. Therefore, in the present specification, "homologous sequence that enables homologous recombination in plastid genome" means an arbitrary base sequence which enables introduction of a promoter by causing homologous recombination.

In the following examples, tobacco plant is transformed, however, the plant to be used is not limited thereto. The technique of plastid transformation is practically applicable to tobacco plants. Moreover, successful examples in Arabidopsis thaliana, potato or tomato are described in some reports. In principle, the method of the present invention can be applied to various plants, such as rape, soybean, rice, etc. Such plants can be also used for the purpose of the present invention, so long as the over-expression of the *accD* operon can be achieved by the method of the present invention.

As a promoter to over-express the *accD* operon, ribosomal RNA operon 16 promoter, used in the example, is preferable. However, the present invention is not limited to the ribosomal RNA operon 16 promoter, other promoters can be also used, so long as they over-express the *accD* operon in plastids and chloroplasts. For example, a promoter such as PSII 32kDa protein (psbA), ribosomal protein S16 (rps16) and the like can be utilized. Thus, in the present specification, "a promoter sequence of a gene exhibiting abundant expression in plastids and chloroplasts" means a nucleotide sequence of an arbitrary promoter, which increases expression of the *accD* operon in plastids and chloroplasts.

As a method of transformation, the particle bombardment is used in the following examples. As a method for plastid transformation, the particle bombardment is the most preferable, for its simplicity and assurance, however, the scope of the invention is not limited thereto. In a successful example, gene was introduced into protoplast using PEG, thereby plastid transformation was achieved. Thus the PEG method can be also utilized, transformation of chloroplast can be achieved using such a method to increase expression of the *accD* gene. Therefore, such embodiment should be also included in the scope of the present invention.

Moreover, a transformed plant, in which the *accD* gene is over-expressed according to the method of the present invention, is also within the scope of the present invention. In the following example, the present inventors have practically produced a transformed plant line. In addition, they have confirmed that seed oil production increases in such transformed line. In the future, the improvement of the present invention will results in increased oil production. Incidentally, in the present specification, the term "control line" means a plant in which *accD* operon is not over-expressed. In the following example, the "cassette introduced line" corresponds to it. Moreover, the term "ratio of fatty acids to starch is improved" means the increased fatty acids accompanied with decreased starch.

Further, by using the method of the present invention, accompanied with promotion of fatty acid synthesis, inhibition in starch accumulation was recognized in the plants. In the other word, by increasing the expression of acetyl-CoA carboxylase, partitioning between starch and fatty acid has been altered, thereby the fatty acid content per leaf or seed increased and the starch content decreased. This finding means that the promotion of the fatty acid synthesis by the method of the present invention has been essentially achieved by alteration in distribution of the product obtained from a determined amount of photosynthesis energy.

Furthermore, as shown in the following examples, increased leaf longevity and increased seed number per plant body were observed in the transformants over-expressing the *accD* operon. The increased seed yield is ascribed to the increased assimilates caused by extended leaf longevity. Over-expression of *accD* operon increases acetyl-CoA carboxylase, which results in abundant supply of fatty acids required for repair of thylakoid membrane. Possibly this supply of fatty acids bring about extended leaf longevity. There is a possibility that increased leaf longevity and increased seed number are caused by abundant expression of *accD* gene together with abundant expression of *psaI, ycf4, cemA* and *petA*. In a transformant over-expressing the *accD* gene, the fatty acid content per seed increased. Therefore, considering the increment in the number of obtained seeds, in the following examples, the fatty acid content per a plant body increases twice or more. The teaching on the gene cluster including *accD, psaI, ycf4, cemA* and *petA* are described in the literatures described below. In the literature of Shinozaki et al (Shinozaki K. et al. (1986) EMBO J. 5, 2043-2049), the entire sequence of tobacco plastid DNA is described. Moreover, transcriptional unit of the gene cluster is described on pease, in which co-transcription of the gene cluster is demonstrated (Nagano Y.et al. (1991) Curr. Genet. 20, 431-435). It is further confirmed on the tobacco plant by Hajdukiewicz P.T.J et al. (Hajdukiewicz P.T.J et al. (1997) EMBO J. 16, 4041-4048).

## EXAMPLES

(Isolation of *accD* gene, *rbcL* gene and *rrn16* promoter)

In this research, *N.tabacum cv. Xanthi* was adopted, for this plant has been successfully applied for plastid transformation. Therefore, the requisite fragments of this cultivative species were cloned. That is, cloning was performed to obtain (1) *accD* gene to be expressed at high extent, (2) *rbcL* (Rubisco large subunit) gene existing adjacent to the *accD* gene and used as a homologous sequence in the homologous recombination, and (3) *rrn* (ribosomal RNA operon) 16 promoter to achieve abundant expression.

Shinozaki et al. (EMBO J.5, 2043-2049 1986) has determined DNA sequence of *N.tabacum cv.* Bright yellow 4 and a primer was designed from this information. Moreover, the respective gene and the promoter were amplified by PCR by using genomic DNA of *Xanthi* as a template. Amplified fragments were subjected to subcloning at the EcoRV site of pZEr0-2.1 (available from Invitrogen) to confirm the sequences.

The sequence of the amplified fragment of *accD* gene was the same as that of bright yellow 4. The sequence of *accD* gene is shown in Fig. 2 and in SEQ.ID.NO:1 of the sequence listing. The sequence of the amplified fragment derived from *rbcL* gene exhibited different in two positions (bases surrounded by the square). The sequence of *rbcL* gene is shown in Fig. 3 and in SEQ.ID.NO:2 of the sequence listing. In the base sequence, some difference was recognized from that described in the known report, however, amino acid encoded from the base sequence was not different from bright yellow 4. This difference is assumed to be due to the difference of the cultivative species of the tobacco plant.

In the meantime, existence of two kinds of *rrn16* promoters has been reported (Curr. Genet. 27, 280-284, 1995). These are, a promoter to which the plastid-encoded plastid RNA polymerase (PEP) is bound and another promoter to which the nuclear-encoded plastid RNA polymerase (NEP) is bound. The sequence of *rrn16* promoter is shown in Fig. 4 and in SEQ.ID.NO:3 of the sequence listing. The *accD* has been known to have a promoter for NEP (Trends Plant Sci., 4, 169-170, 1999). In this experiment, both promoters of *rrn16* were amplified. The sequences of the amplified fragments were the same as that of bright yellow 4.

(Preparation of construct)

A construct for plastid transformation was prepared. As the expression of *accD* gene is low, the inherent promoter was removed and recombined by the potent *rrn16* promoter. Moreover, as spectinomycin is utilized for selection of the transformant, both of the drug resistant gene and *aadA* (aminoglycoside 3''-adenyltransferase) gene were introduced together. Here, spectinomycin inhibits protein synthesis in chloroplast. Moreover, to confirm that insertion of *aadA* gene did not cause any damage to the plant body, a control construct was also prepared and only *aadA* gene was inserted into the construct.

The structure of the construct, utilized to recombine the promoter of *accD* gene, is shown in Fig. 5. The inherent promoter of *accD* gene was removed and it was replaced by *rrn16* promoter. The *aadA* gene was inserted between *rbcL* gene and *accD* gene. The promoter for PEP of *rrn16* was ligated to the upstream of the *addA* gene and the terminator of *psbA* (PS11 32kDa protein) was ligated to the downstream of *addA* gene (referred to aadA cassette). The construct used to insert only *aadA* gene is shown in Fig. 6. The aadA cassette was inserted into upstream of the inherent *accD* promoter.

Concrete method for production of the construct is described below. The coding region of *rbcL* gene and the downstream region were amplified. The forward primer (5'-GTCGAGTAGACCTTGTTGTTGTGAG-3': SEQ.ID.NO:4 of the sequence listing) and the reverse primer (5'-CCCGGGCGGCCGCGGAACCCCCTTTATATTAT-3': SEQ.ID.NO:5 of the sequence listing) were used. The reverse primer contains restriction sites of SmaI, NotI and SacII at the 5' side. The fragment amplified by the primer was subjected to subcloning at the EcoRV site of pzErO-2.1 (available from Invitrogen) and digested with EcoRI and SmaI. The DNA fragments were separated by electrophoresis and the objective DNA fragment was recovered by DNA extraction kit (available from Amasham Pharmacia). The region corresponding to -18 to +86 (the transcription initiation site was used as the criteria of the numbering) was removed from *accD* gene and the region corresponding to +87 to 1674 was amplified. The forward primer (5'-CCGCGGCCGCCCGGGGTCTGATAGGAAATAAG-3': SEQ.ID.NO:6 of the sequence listing) and the reverse primer (5'-GTCGACGTGCTCTACTTGATTTTGC-3': SEQ.ID.NO:7 of the sequence listing) were used. The forward primer contained SacII, NotI and SmaI sites at the 5' end and the reverse primer contained SalI site at the 5' end. The amplified fragment was subjected to subcloning to the EcoRV site of pzErO-2.1 (available from Invitrogen) and digested by SmaI and SalI.

The DNA fragments were separated by electrophoresis and the objective fragment was recovered by DNA extraction kit. This fragment was ligated to pUC18 digested by SmaI and SaI. This plasmid was digested by EcoRI and SmaI, then ligated to the above-mentioned fragment, which was amplified and recovered fragment. Thus the objective plasmid was prepared and the plasmid contained *rbcL* gene and *accD* gene with the promoter region removed. The *rrn16* promoter was amplified using the forward primer (5'-GTCGACGCTCCCCCGCCGTCGTTC-3': SEQ.ID.NO:8 of the sequence listing) and the reverse primer (5'-GGTACCCGGGATTCGGAATTGTCTTTC-3': SEQ.ID.NO:9 of the sequence listing). The forward primer contained the SalI site at the 5' end and the reverse primer contained the KpnI and SmaI sites at the 5' end. The amplified fragment was subjected to subcloning at the EcoRV site of pZErO-2.1 and digested by SalI and KpnI. Then the DNA fragment was separated by electrophoresis by using a low melting point agarose gel, the objective DNA fragment was extracted with phenol, and it was recovered. This fragment was ligated to pCT08 vector containing the aadA cassette digested by SalI and KpnI, then a plasmid containing aadA cassette and *rrn16* promoter was prepared. Finally, the plasmid containing aadA cassette and *rrn16* promoter was digested by SmaI and NotI, then the DNA fragment was separated by electrophoresis and the DNA fragment was recovered by DNA extraction kit. This fragment was ligated to the plasmid, containing *rbcL* gene digested by SmaI and NotI and *accD* gene with the promoter region removed. Then the objective construct was completed (Fig. 5).

A construct used to introduce only *aadA* gene was prepared as follows. Using a forward primer (5'-GTCGACAACATATTAATATATAGTG-3': SEQ.ID.NO:10 of the sequence listing) and a reverse primer (5'-GTCGACGTGCTCTACTTGATTTTGC-3': SEQ.ID.NO:11 of the sequence listing), the *accD* gene with the inherent *accD* promoter was amplified. The forward primer contained SalI site at the 5' end and the reverse primer contained SalI site at the 5' end. The amplified fragment was subjected to subcloning at the EcoRV site of pZErO-2.1 and digested by SalI. The DNA fragment was separated by electrophoresis and the objective DNA fragment was recovered by DNA extraction kit. The donor DNA shown in Fig. 5 was digested by SalI and the *accD* gene having *rrn16* promoter was removed. The resultant plasmid was ligated to *accD* gene having the inherent promoter to complete the construct (Fig. 6). The total sequences of each construct were confirmed, purified by Quiagen column, then the constructs were used for the plastid transformation.

(Transformation of chloroplast)

Transformation of chloroplast gene was performed according to the system developed by Maliga et al. (Proc. Natl. Acad. Sci. USA; 90, 913-917, 1993). Sterilized tobacco seeds were germinated in RM medium (MS salts, 30 g/l sucrose, 6 g/l Agar) containing no drug and they were grown for 6 to 8 weeks in an artificial weather conditioner. The growing condition was the temperature of 25°C, the illumination intensity of 40 μmol/m$^2$. s and the illumination cycle of 16 hours in the light and 8 hours in the dark. The plants were grown to the height of the agricultural pot and the third or fourth leaves from the top of the plant bodies were used for transformation. The leaves were placed on a RMOP medium (MS salts, 0.005% FeEDTA, 1 mg/1T hiamine, 0.1 ml/l NAA, 1 mg/l BAP, 100 mg/l inositol, 30 g/l suctose, and 6 g/l Agar) with their back side up. The vein and peripheral portion of the leaves were cut and the leaves were completely spread on the filter paper underlying the medium. Under this condition, they were allowed to stand at 25°C for overnight in a clean bench. Tungsten particles (1.1 μm) were used in the bombardment of particle gun. They were treated by ethanol at 95°C for 2 hours, then subjected to sonication treatment. Subsequently ethanol was removed and they were suspended in sterilized water. Fifty μl of the tungsten solution, the prepared construct corresponding to 10 μg, 50 μl of 2.5 mM calcium chloride and 20 μl of 0.1 mM spermidine were mixed and suspended at 4°C for 30 minutes.

Then, the mixture was washed three times with ethanol, and the particles were suspended in 30 μl of ethanol. Then they were utilized for 5 times of bombardments. The tobacco leaves were left overnight and 10 sheets of leaves were bombarded by the each construct (one leaf was used for one bombardment). As the particle gun, PDS1000He manufactured by Bio-Rad was used. The sample was set at the fifth stage and a stopping screen was set at the second stage. After reducing the atmosphere to reach the vacuum pressure of 27.5, bombardment was performed using rupture disc of 1100 psi. After the bombardment, the atmosphere was immediately returned to the ordinal pressure and the leaves were incubated for 2 days in an artificial weather conditioner under the following condition. The condition was the temperature of 25°C, the illumination intensity of 40 μmol/m$^2$. s and the illumination cycle of 16 hours in the light 8 hours in the dark. Thereafter, the leaves were cut into squares of 5 mm and they were planted in the PMOP medium containing 50 mg/l of spectinomycin.

Some individuals formed green shoots within approximately one month and such individuals were transplanted to the RMOP medium containing 500ml/l of spectinomycin and they were allowed to take roots. As to those failed to take roots, leaves were cut from such individuals, planted to the RMOP medium containing spectinomycin and they were again subjected to regeneration. The formed shoots were transplanted to the RMOP medium containing spectinomycin and they were allowed to radicate. As to individuals radicated within 1 to 2 weeks, they were grown to the height of the agricultural pot, then several leaves were cut from the individuals. Then genomic DNA was extracted from them and the inserted gene was confirmed by PCR. As to the auxiliary buds derived from the individuals with gene introduction, they were transplanted to the RMOP medium containing spectinomycin to increase the number of the individuals. In this research, several lines were obtained on the transformants with the *accD* promoter recombined (line with abundant expression of the *accD* gene) and on the transformants wherein only *aadA* gene was introduced (cassette introduced line).

(Genomic southern blot analysis of the transformed plants)

Meanwhile, a great number of copies exist in plastid genome and the number in a cell reaches as many as 50 to 10,000 copies. In the plastid transformation, it is difficult to recombine all of the plastid genome. Therefore, in the transformants, it is likely that the wild type and the transformed plastid genome both exist in an admixture. Thus, on the resultant transformants, the ratio between the wild type and the transformed plastid genomes was examined by genomic southern analysis.

Individuals grown in an agricultural pot were transplanted in soil and they were grown without drugs in a green house at the temperature of 25°C, the luminance intensity of 300 μmol/m$^2$ and the illumination cycle of 12 hours in the light and 12 hours in the dark. When 3 or 4 leaves were formed, using several lines of individuals, genomic DNA was extracted from approximately 500 mg of leaves by the CTAB (cetyltrimethyl ammonium bromide) method. Two μg of this genomic DNA was digested by EcoRI, then it was subjected to electrophoresis by 0.8% of agarose gel. The gel was subjected to the acid treatment, then to the alkaline treatment. Subsequently, it was transferred to Hybond N+ membrane (available from Amasham Pharmacia). The transferred membrane was pretreated at 65°C for one hour by hybridization solution containing 1% (w/v) PVP K30, 1mM EDTA, 500mM sodium phosphate buffer (pH 7.2) and 7% (w/v) SDS, then the $^{32}$P-labeled probe was added to the solution to achieve the radio activity of 2x10$^6$ cpm/ml. Hybridization was carried out at 65°C for 16 hours. The fragment containing full-length *accD* and *aadA* genes was labeled with α-$^{32}$P dCTP, using BcaBest labeling kit (TAKARA). After hybridization, the membrane was washed at room temperature for 15 minutes with 2 x SSPE (1 x SSPE: 0.15M NaCl, 10mM $NaH_2PO_4$ [pH7.4], 1mM EDTA) containing 0.1% SDS. After washing twice, it was exposed to Fuji Imaging Plate for overnight, then the signals were detected by BAS2000 imaging analyzer (manufactured by Fuji Film Co.).

In the wild-type line, *accD* gene was detected at approximately 4.3 kbp (Fig. 7.). In the control line (cassette introduced line) and in the line with *accD* abundant expression, only a band of 5.7 kbp was detected. The molecular size of the band was about 1.4 kbp larger than hat of the wild-type line, indicating that the drug-resistant gene marker was inserted. Thus, it was confirmed that homologous recombination occurred only on the objective position. Moreover, when PCR analysis was performed using a primer that amplifies the wild-type plastid genome, no amplified fragment was detected. Therefore, complete recombination of the plastid genome was confirmed and *aadA* gene was not detected in the wild-type line. In the control line and in the line with *accD* abundant expression, the band was detected at 5.7 kbp. Thus, insertion of the *aadA* gene was also confirmed (Fig. 8).

(Northern blotting analysis of the transformed plant)

The promoter of the *accD* gene was replaced with the *rrn16* promoter for abundant expression. Then the northern blot analysis was performed to examine expression of the *accD* gene. Analysis was performed using a plant body having 9 leaves. The leaves existing at the second to the fourth from the top were collected from wild-type line, the control line and the line with *accD* abundant expression, respectively. Then nucleic acid was extracted by the SDS-phenol method. From the resulting nucleic acid fraction, total RNA was recovered by LiC1 precipitation method. For each lane, total RNA corresponding to 1 μg was subjected to electrophoresis by 1% agarose gel containing 0.66M formaldehyde. After the electrophoresis, the gel was transferred to Hybond N+ membrane. The transferred membrane was pretreated at 42°C for one hour in hybridization solution containing 50% (v/v) formaldehyde, 6 x SSPE, 0.5% (w/v) SDS, 0.1 mg/ml degenerated salmon testis DNA, and 5% (v/v) irish cream. Then, the $^{32}P$-labelled probe was added to the solution to achieve radio activity of $2x10^6$ cpm/ml and hybridization was performed at 42°C for 16 hours. The probe was prepared by $\alpha$-$^{32}P$ dCTP labeling of the fragment containing full-length *accD* gene, using BcaBest labeling kit. After the hybridization, the membrane was washed twice at room temperature for 15 minutes with 2XSSPE containing 0.1% SDS, and then washed twice at 65°C for 30 minutes with 2XSSPE containing 0.1% SDS. After washing, the hybridized membrane was exposed to Fuji Imaging Plate for overnight and the signals were detected by BAS2000 Imaging Analyzer (manufactured by Fuji Film Co.).

In all of the lines including wild-type line, the control line and the line with *accD* abundant expression, the transcription product of 2.3 kb was detected (Fig. 9). This was accorded with the size of tobacco *accD* mRNA, which has already reported (EMBO J.16, 4041-4048, 1997). The amount of mRNA was low in the wild-type line and it slight increased in the control line. On the other hand, in the line with *accD* abundant expression, the expression increased significantly.

Then, the inventors succeeded to increase *accD* mRNA, by recombination of the inherent promoter of the *accD* gene with a promoter that enable abundant expression of the *accD* gene.

(Western blot analysis of the transformed plant)

As the amount of *accD* mRNA existing in the plastid genome increased, the inventors analyzed on the amount of CTβ (carboxytransferase beta subunit) to examine whether the translation product increased or not. Simultaneously, the three nuclear-encoded subunits of CTα (carboxytransferase alpha subunit), BC (biotin carboxylase) and BCCP (biotin carboxyl carrier protein) were also examined. To achieve the purpose of this invention, it is necessary to finally increase the amount of acetyl CoA carboxylase (ACCase). To increase ACCase, it is requisite to increase in the amounts of all subunits. For it is difficult to measure ACCase activity, western blot was performed to analyze the amount of the respective subunit.

Analysis was performed using plant bodies having 9 leaves. The leaves existing at the second to the fourth from the top were collected from the wild-type line, the control line and the line with *accD* abundant expression, respectively. They were crushed in S-300 buffer (50mM Tris-HCl [pH 8.0], 5mM aminocaproic acid, 1mM EDTA, 1mM Benzamidine, 20mM β-mercaptoethanol, and 1mM PMSF) with addition of aluminum oxide and proteins were extracted. Then, an aliquot of 2xSDS loading buffer (6% SDS, 125 mM Tris-HCl [pH 6.8] and 20% glycerol) was added. After heating at 99°C for 3 minutes, the mixture was centrifuged and the supernatant was recovered. The DC Protein Assay (BIO-RAD), which accorded to the Lowry method, was used for quantification of the protein. At the SDS-PAGE analysis, separation was performed with addition of β-mercaptoethanol and BPB (separation gel concentration of 10%). To each lane, 10 μg of protein was applied. After electrophoresis, the gel was transferred to the nitrocellulose membrane. Then the membrane was subjected to blocking and reacted with the respective polyclonal antibodies (the anti-CTβ antibody was used at 1/1000 dilution,. the anti-BC and the CT antibodies were used at 1/3000 dilution). After the reaction, the membrane was washed and reacted with the secondary antibody (an anti-rabbit antibody labeled by HRP) diluted to 1/3000. Finally, the membrane was washed and the signal was detected using ECL kit (available from Amasham Pharmacia). The membrane was transferred and blocked, then BCCP was reacted with HRP-labelled streptoavidin diluted to 1/1000. The membrane was washed and the signal was detected using ECL kit.

The amount of CTβ increased in the line with *accD* abundant expression, compared with that of the wild-type line (Fig. 10). Moreover, all of the nuclear-encoded subunit proteins (BC, BCCP and CTα) increased in the line with *accD* abundant expression, compared with that of the wild-type line. All of the subunits increased in the line with *accD* abundant expression, therefore, the inventors finally succeeded in increaseing the amount of plastidic acetyl CoA carboxylase.

(Gas chromatography analysis of fatty acid content)

The inventors further investigated on whether the increased ACCase leads to promotion of fatty acid synthesis, which results in increased oil content in seeds. Using the seeds obtained from the wild line, the control line and the line with *accD* abundant expression, the amounts of fatty acids were analyzed by gas chromatography, as the fatty acids were the main component of oil.

To 50 seeds derived from each line, 250 μl of 5% HCl-methanol was added, then treated at 80°C for 1.5 hours. By this procedure, the fatty acid was altered to its methyl ester. After the reaction, 1.5 ml of 0.9% (w/v) sodium chloride and 0.5 ml of hexane were added to extract the methylated fatty acids. The hexane layer was recovered and 1 μl of the solution was used for the gas chromatography analysis. As the device of the gas chromatography analysis, GL-353 (manufactured by GL Sciences Inc.) was used and CP-si188 (0.25mmx50m) was used as capillary column. Analysis was performed under the column pressure of 160 kPa, the temperature of 190°C and the analysis time of 15 minutes.

In tobacco seeds, fatty acids of C16:0, C18:0, C18:1, C18:2 and c18:3 were detected. The amounts of fatty acids were calculated from the detected peaks and the fatty acids contents per a dried seed were compared (Table 1). The fatty acid content in the seeds of the wild-type line was 42.5 mg/g dry weight in average. In the seeds of the control line, the amount was 43.3 mg/g dry weight in average, which is almost equal to that of the wild-type line. On the other hand, in seeds of the line with *accD* abundant expression, the amount was 47.6 mg/g dry weight in average. Therefore, the fatty acid content increased 12%, compared with the wild-type line. The line D exhibited the maximum fatty acid content and the fatty acid content increased 22% in this line D. In a colza plant, wherein the eukaryotic form enzyme is abundantly expressed by introduction of the chloroplast transition signal attached to the eukaryotic type ACCase, 5% of fatty acid increase was confirmed. Therefore, the line D exhibited higher result, compare with this plant with abundant expression of eukaryotic form ACCase. That is, the present invention provided a plant exhibiting abundant expression of *accD* accompanied with increase in the ACCase, thereby the seed oil was accumulated in this plant.

Table 1

| | Total fatty acid (mg/g dry weight) |
|---|---|
| Wild-type line A | 43.6 |
| Wild-type line B | 41.4 |
| Control line A | 43.3 |
| Control line B | 43.2 |
| Line with accD abundant expression A | 45.1 |
| Line with accD abundant expression B | 48.3 |
| Line with accD abundant expression C | 45.0 |
| Line with accD abundant expression D | 51.8 |

(Phenotype of the transformant)

Using plants having both promoters of *rrn16* instead of the inherent *accD* promoter, the phenotype of the plants was observed. As to the growth rate, the length of the plant body and the size of the leaves, no difference was observed on these traits compared to the wild-type line and the control line. However, difference was observed in aging of leaves (Fig. 11). In the wild-type line and in the control line at the growth stage (12 weeks old), leaves at the bottom portion were aged and the color turned to yellow (Fig. 11, arrows of the wild-type line and the control line). In the line with abundant expression of *accD*, the leaves remained its green color (Fig. 11, the arrow of the line with *accD* abundant expression) and the beginning of the leaf aging delayed 7 to 10 days. Thus, the life longevity of the leaves was extended in the line with *accD* abundant expression.

(Observation of chloroplast of the transformed plant)

In the line with abundant expression of accD, the amount of ACCase increased and life longevity of the leaves was extended. In view of these findings, the chloroplast of the leaf was observed by electric microscope. A matured leaf of a plant at 12 weeks old was fixed with 50mM sodium phosphate buffer (pH 7.2) containing 5% (v/v) glutaraldehyde for 8 hours. After this fixation, post-fixation treatment was performed with 2% (v/v) osmium tetraoxide solution. Then, the fixed material was dehydrated with a series of acetone and encapsulated in Epon 812 resin. The ultra-thin slice was stained by 2% uracil acetate and plumbum citrate, then observed by electric microscope. The results in the wild-type line and in the line with accD abundant expression are shown in Figs. 12 and 13, respectively. In the chloroplast of leaves derived from the line with accD abundant expression, the size of the starch granules became smaller (Fig. 13, black arrow) and many oil droplets (Fig. 13, white arrow) were observed. Therefore, in accordance with increase of ACCase, the starch content decreased and the oil content increased.

(Analyses of fatty acid and starch contents in leaves)

To confirm decreased starch content and increased oil content in the leaves derived from the line with accD abundant expression, chemical analysis was performed on fatty acid and starch contents. The amount of the fatty acid, which is a main component of oil, was analyzed as mentioned below. To tissue pieces (four sheets of tissue pieces having the size of 4.8mm) of leaves derived from each line, 250 μl of 5% hydrochloric acid-methanol was added and it was treated at 80°C for 1.5 hours. By this procedure, fatty acids were converted to their methyl esters. After the reaction, 1.5 ml of 0.9% (w/v) sodium chloride and 0.5 ml of hexane were added to extract the methylated fatty acids. The hexane layer was recovered and 1 μl of the solution was used for the gas chromatography analysis. GL-353 (manufactured by GL Sciences Inc.) was used for this analysis. In addition, as a capillary column, CP-si188 (0.25mmx50m) was used. Analysis was performed at the column pressure of 160 kPa, the temperature of 190°C and the analysis time of 15 minutes.

The starch content was analyzed as mentioned below. Tissue pieces (one sheet of tissue piece having a size of 4.8mm) of the leaves derived from each line were crushed in 80% ethanol and heated at 80°C for one hour. This operation was repeated twice. To the residue, 0.2N sodium hydroxide solution was added and heated at 95°C for one hour. Then the mixture was neutralized with acetic acid and the supernatant was recovered (referred to starch fraction). The starch fraction was treated by α-amylase (pH 5.2, 37°C, 30 minutes) and amyloglucosidase (pH 4.6, 55°C, one hour), thereby the starch was decomposed to monosaccharides. Then, this monosaccharide was reacted with glucose 6-phosphate dehydrogenase and hexokinase at 30°C in reaction buffer (100mM imidazole, 1.5mM magnesium chloride, 1.1mM ATP and 0.5 mM â-NADP+). Absorbance at 340 nm was measured and the amount of starch was calculated from the value. For preparation of a calibration curve, glucose solution was used. They were analyzed four times (twice analyzed for two lines) and the average value was calculated on both of fatty acid and starch contents.

In tobacco leaves, fatty acids of C16:0, C18:0, C18:1, C18:2 and c18:3 were detected. From the detected peaks, the amount of fatty acid was calculated and the fatty acid content per fresh weight was compared. The starch content was also compared by the content per fresh weight. The fatty acid content was 3.9 μg/mg (fresh weight) and the starch content was 182 μg/mg (fresh weight), for the control line. These values were calibrated to 1.0 and the results were indicated by the relative value obtatined by the calibration. The results of the fatty acid contents and starch contents are shown in Figs. 14 and 15, respectively. In the line with accD abundant expression, the fatty acid content increased about 10% and the starch content decreased about 7%. Accompanied with increase of ACCase, the content of starch decreased and the content of fatty acid increased in the leaves.

(Analyses of fatty acid content and starch content in seeds)

In accordance with the extended life longevity of the leaves derived from the line with accD abundant expression, the period of photosynthesis might be extended as well. As a result, the number of the seed might increase, for the seeds are storage organization. Furthermore, caused by increase of ACCase, the starch content might increase and the fatty acid content might decrease. Thus, fatty acid and starch contents per a seed, as well as per a plant body, were analyzed.

All of the seeds were collected from the respective lines, and the collected 50 seeds were used for analysis of fatty acid content. The analysis was performed in the same manner as described for the leaves. In the seeds, fatty acids of C16:0, C18:0, C18:1, C18:2 and c18:3 were detected. The fatty acid contents were calculated from the detected peaks. The amount of starch was quantified using 100 seeds, according to the method of Starch assay kit (available from Sigma). Both of the fatty acid and the starch contents were analyzed three times for one line. The control line was analyzed on three individuals, the line A with accD abundant expression was analyzed on four individuals and the line B with accD abundant expression was analyzed on three individuals. Then the average values were obtained.

The fatty acid and the starch contents per dried 1000 seeds of the control line were 3.8 mg and 0.4 mg, respectively. These values were calibrated to 1.0 and the results were compared. The results of the fatty acid contents and starch contents are shown in Figs. 16 and 17, respectively. In the seeds derived from the line with accD abundant expression, fatty acid content increased approximately 40% and starch content decreased approximately 20%. As shown in the leaves, accompanied with increase of ACCase, decrease in the starch content and increase in the fatty acid content, were also observed on the seeds, as well.

Next, the fatty acid and starch contents per a plant body were compared. The results of the fatty acid contents, starch contents are shown in Figs. 18 and 19, respectively. The result examined on partitioning is shown in Fig. 20. The average numbers of seeds obtained per a plant body were 5200 (the average of three individuals) for the control line, 13000 (the average of four individuals) for the A1 line with accD abundant expression and 9000 (the average of three individuals) for the A2 line. In the line with accD abundant expression, the number of the obtained seeds increased 1.7 to 2.5 times. The contents per a plant body were also calculated from these values and the total contents of fatty acid increased 2.3 to 3.3 times. Moreover, the total contents of starch increased 1.4 to 1.9 times. When the fatty acid/starch ratio was compared in the whole plant body, the ratio of fatty acid increased in the line with accD abundant expression. Due to increase of ACCase, the partitioning of starch and fatty acid was altered, thereby the fatty acid content per a seed increased. Moreover, the life longevity of the leaves was extended and the number of seeds obtained per a plant body increased. Therefore, the inventors finally succeeded in increasing fatty acid content per a plant body. Considering that almost all the fatty acids are stored as oils in seeds, the oil content increased significantly.

Moreover, in the present invention, a plant with accD abundant expression was provided and the amount of ACCase increased in the plant. In addition, life longevity of the leaves was extended in the plant, thereby the period of photosynthesis is extended in the plant This phenomenon lead to increase in the number of obtained seeds, which is a storage organ. Moreover, due to increase of ACCase, fatty acids synthesis was activated and that of starch was inhibited. That is, partitioning of the photosynthesis product was altered in the plant. According to these results, the inventors succeeded in increasing the fatty acid content per a plant. If further improvement will be achieved in the future, further increase in the fatty acid content will be enabled by the present invention. The method of this invention is a novel technique applicable to crops, garden plants, foliage plants and the like.

According to the present invention, a novel method for promoting fatty acid content in a plant is provided. In this method, the promoter of *accD* gene of acetyl-CoA carboxylase was replaced with a promoter directing abundant expression in plastids and chloroplasts by using plastid transformation. This method increases the carboxyltransferase beta subunit protein encoded by the *accD* gene. Accompanied with it, the other subunits constituting the acetyl-CoA carboxylase apparently increases and this enzyme increases. Since the acetyl-CoA carboxylase is a key enzyme for rate-limiting step of fatty acid synthesis, synthesis of fatty acid can be promoted by the method of the present invention. The transformed plant produced according to the method of the present invention exhibits remarkable enhancement in the fatty acid content in leaves and seeds. Moreover, the leaf longevity extended and the number of the seeds per a plant body increased, thereby seed oil and productivity of the plant are improved.

SEQUENCE LISTING

<110> NARA INSTITUTE OF SCIENCE AND TECHNOLOGY

<120> A METHOD FOR PROMOTING FATTY ACID SYNTHESIS IN A PLANT

<160> 11

<210> 1

<212> 1830

<212> Nucleic acid

<213> *N. tabacum cv.Xanthi*

<400> 1

CGAATTTGAC ACGACATAGG AGAAGCCGCC CTTTATTAAA AATTATATTA TTTTAAATAA 60
TATAAAGGGG GTTCCAACAT ATTAATATAT AGTGAAGTGT TCCCCCAGAT TCAGAACTTT 120
TTTTCAATAC TCACAATCCT TATTAGTTAA TAATCCTAGT GATTGGATTT CTATGCTTAG 180
TCTGATAGGA AATAAGATAT TCAAATAAAT AATTTTATAG CGAATGACTA TTCATCTATT 240
GTATTTTCAT GCAAATAGGG GGCAAGAAAA CTCTATGGAA AGATGGTGGT TTAATTCGAT 300
GTTGTTTAAG AAGGAGTTCG AACGCAGGTG TGGGCTAAAT AAATCAATGG GCAGTCTTGG 360
TCCTATTGAA AATACCAATG AAGATCCAAA TCGAAAAGTG AAAAACATTC ATAGTTGGAG 420
GAATCGTGAC AATTCTAGTT GCAGTAATGT TGATTATTTA TTCGGCGTTA AAGACATTCG 480
GAATTTCATC TCTGATGACA CTTTTTTAGT TAGTGATAGG AATGGAGACA GTTATTCCAT 540
CTATTTTGAT ATTGAAAATC ATATTTTTGA GATTGACAAC GATCATTCTT TTCTGAGTGA 600
ACTAGAAAGT TCTTTTTATA GTTATCGAAA CTCGAATTAT CGGAATAATG GATTTAGGGG 660
CGAAGATCCC TACTATAATT CTTACATGTA TGATACTCAA TATAGTTGGA ATAATCACAT 720
TAATAGTTGC ATTGATAGTT ATCTTCAGTC TCAAATCTGT ATAGATACTT CCATTATAAG 780
TGGTAGTGAG AATTACGGTG ACAGTTACAT TTATAGGGCC GTTTGTGGTG GTGAAAGTCG 840
AAATAGTAGT GAAAACGAGG GTTCCAGTAG ACGAACTCGC ACGAAGGGCA GTGATTTAAC 900
TATAAGAGAA AGTTCTAATG ATCTCGAGGT AACTCAAAAA TACAGGCATT TGTGGGTTCA 960
ATGCGAAAAT TGTTATGGAT TAAATTATAA GAAATTTTTG AAATCAAAAA TGAATATTTG 1020
TGAACAATGT GGATATCATT TGAAAATGAG TAGTTCAGAT AGAATTGAAC TTTTGATCGA 1080
TCCGGGTACT TGGGATCCTA TGGATGAAGA CATGGTCTCT CTAGATCCCA TTGAATTTCA 1140
TTCGGAGGAG GAGCCTTATA AAGATCGTAT TGATTCTTAT CAAAGAAAGA CAGGATTAAC 1200
CGAGGCTGTT CAAACAGGCA TAGGCCAACT AAACGGCATT CCCGTAGCAA TTGGGGTTAT 1260
GGATTTTCAG TTTATGGGGG GTAGTATGGG ATCCGTAGTC GGAGAGAAAA TCACCCGTTT 1320
GATTGAATAC GCTGCCAATC AAATTTTACC CCTTATTATA GTGTGTGCTT CTGGGGGGGC 1380
GCGCATGCAG GAAGGAAGTT TGAGCTTGAT GCAAATGGCT AAAATATCGT CTGCTTTATA 1440

TGATTATCAA TTAAATAAAA AGTTATTTTA TGTATCAATC CTTACATCTC CGACAACTGG 1500
TGGAGTGACA GCTAGTTTTG GTATGTTGGG GGATATCATT ATTGCCGAAC CCAACGCCTA 1560
CATTGCATTT GCAGGTAAAA GAGTAATTGA ACAAACATTG AATAAAACAG TACCCGAAGG 1620
TTCACAAGCA GCTGAATACT TATTCCAGAA GGGTTTATTC GACCTAATTG TACCACGTAA 1680
TCTTTTAAAA AGCGTTCTGA GTGAGTTATT TAAGCTCCAC GCCTTTTTTC CTTTGAATCA 1740
AAAGTCAAGC AAAATCAAGT AGAGCACTAA GTTCAATTAT TTTATTTGTG TTTGTAGCAA 1800
AAAAGTAGTT AGTTTGTCGG AATCAAAGTA 1830

<210> 2

<212> 2150

<212> Nucleic acid

<213> *N. tabacum cv. Xanthi*

<400> 2

GTCGAGTAGA CCTTGTTGTT GTGAGAATTC TTAATTCATG AGTTGTAGGG AGGGATTTAT 60
GTCACCACAA ACAGAGACTA AAGCAAGTGT TGGATTCAAA GCTGGTGTTA AAGAGTACAA 120
ATTGACTTAT TATACTCCTG AGTACCAAAC CAAGGATACT GATATATTGG CAGCATTCCG 180
AGTAACTCCT CAACCTGGAG TTCCACCTGA AGAAGCAGGG GCCGCGGTAG CTGCCGAATC 240
TTCTACTGGT ACATGGACAA CTGTATGGAC CGATGGACTT ACCAGCCTTG ATCGTTACAA 300
AGGGCGATGC TACCGCATCG AGCGTGTTGT TGGAGAAAAA GATCAATATA TTGCTTATGT 360
AGCTTACCCT TTAGACCTTT TTGAAGAAGG TTCTGTTACC AACATGTTTA CTTCCATTGT 420
AGGTAACGTA TTTGGGTTCA AAGCCCTGCG CGCTCTACGT CTGGAAGATC TGCGAATCCC 480
TCCTGCTTAT GTTAAAACTT TCCAAGGTCC GCCTCATGGG ATCCAAGTTG AAAGAGATAA 540
ATTGAACAAG TATGGTCGTC CCCTGTTGGG ATGTACTATT AAACCTAAAT TGGGGTTATC 600
TGCTAAAAAC TACGGTAGAG CTGTTTATGA ATGTCTTCGC GGTGGACTTG ATTTTACCAA 660
AGATGATGAG AACGTGAACT CACAACCATT TATGCGTTGG AGAGATCGTT TCTTATTTTG 720
TGCCGAAGCA CTTTATAAAG CACAGGCTGA AACAGGTGAA ATCAAAGGGC ATTACTTGAA 780
TGCTACTGCA GGTACATGCG AAGAAATGAT CAAAAGAGCT GTATTTGCTA GAGAATTGGG 840
CGTTCCGATC GTAATGCATG ACTACTTAAC GGGGGGATTC ACCGCAAATA CTAGCTTGGC 900
TCATTATTGC CGAGATAATG GTCTACTTCT TCACATCCAC CGTGCAATGC ATGCGGTTAT 960
TGATAGACAG AAGAATCATG GTATCCACTT CCGGGTATTA GCAAAAGCGT TACGTATGTC 1020
TGGTGGAGAT CATATTCACT CTGGTACCGT AGTAGGTAAA CTTGAAGGTG AAAGAGACAT 1080
AACTTTGGGC TTTGTTGATT TACTGCGTGA TGATTTTGTT GAACAAGATC GAAGTCGCGG 1140
TATTTATTTC ACTCAAGATT GGGTCTCTTT ACCAGGTGTT CTACCCGTGG CTTCAGGAGG 1200
TATTCACGTT TGGCATATGC CTGCTCTGAC CGAGATCTTT GGGGATGATT CCGTACTACA 1260

GTTCGGTGGA GGAACTTTAG GACATCCTTG GGGTAATGCG CCAGGTGCCG TAGCTAATCG 1320
AGTAGCTCTA GAAGCATGTG TAAAAGCTCG TAATGAAGGA CGTGATCTTG CTCAGGAAGG 1380
TAATGAAATT ATTCGCGAGG CTTGCAAATG GAGCCCGGAA CTAGCTGCTG CTTGTGAAGT 1440
ATGGAAAGAG ATCGTATTTA ATTTTGCAGC AGTGGACGTT TTGGATAAGT AAAAACAGTA 1500
GACATTAGCA GATAAATTAG CAGGAAATAA AGAAGGATAA GGAGAAAGAA CTCAAGTAAT 1560
TATCCTTCGT TCTCTTAATT GAATTGCAAT TAAACTCGGC CCAATCTTTT ACTAAAAGGA 1620
TTGAGCCGAA TACAACAAAG ATTCTATTGC ATATATTTTG ACTAAGTATA TACTTACCTA 1680
GATATACAAG ATTTGAAATA CAAAATCTAG AAAACTAAAT CAAAATCTAA GACTCAAATC 1740
TTTCTATTGT TGTCTTGGAT CCACAATTAA TCCTACGGAT CCTTAGGATT GGTATATTCT 1800
TTTCTATCCT GTAGTTTGTA GTTTCCCTGA ATCAAGCCAA GTATCACACC TCTTTCTACC 1860
CATCCTGTAT ATTGTCCCCT TTGTTCCGTG TTGAAATAGA ACCTTAATTT ATTACTTATT 1920
TTTTTATTAA ATTTTAGATT TGTTAGTGAT TAGATATTAG TATTAGACGA GATTTTACGA 1980
AACAATTATT TTTTTATTTC TTTATAGGAG AGGACAAATC TCTTTTTTCG ATGCGAATTT 2040
GACACGACAT AGGAGAAGCC GCCCTTTATT AAAAATTATA TTATTTTAAA TAATATAAAG 2100
GGGGTTCCAA CATATTAATA TATAGTGAAG TGTTCCCCCA GATTCAGAAC 2150

<210> 3
<212> 220
<212> Nucleic acid
<213> *N. tabacum cv. Xanthi*
<400> 3

GCCCAATGTG AGTTTTTCTA GTTGGATTTG CTCCCCCGCC GTCGTTCAAT GAGAATGGAT 60
AAGAGGCTCG TGGGATTGAC GTGAGGGGGC AGGGATGGCT ATATTTCTGG GAGCGAACTC 120
CGGGCGAATA TGAAGCGCAT GGATACAAGT TATGCCTTGG AATGAAAGAC AATTCCGAAT 180
CCGCTTTGTC TACGAACAAG GAAGCTATAA GTAATGCAAC 220

<210> 4
<212> 25
<212> Nucleic acid
<213> Artificial Sequence
<400> 4

GTCGAGTAGA CCTTGTTGTT GTGAG

<210> 5
<212> 32
<212> Nucleic acid

<213> Artificial Sequence

<400> 5

CCCGGGCGGC CGCGGAACCC CCTTTATATT AT

<210> 6

<212> 32

<212> Nucleic acid

<213> Artificial Sequence

<400> 6

CCGCGGCCGC CCGGGGTCTG ATAGGAAATA AG

<210> 7

<212> 25

<212> Nucleic acid

<213> Artificial Sequence

<400> 7

GTCGACGTGC TCTACTTGAT TTTGC

<210> 8

<212> 24

<212> Nucleic acid

<213> Artificial Sequence

<400> 8

GTCGACGCTC CCCCGCCGTC GTTC

<210> 9

<212> 27

<212> Nucleic acid

<213> Artificial Sequence

<400> 9

GGTACCCGGG ATTCGGAATT GTCTTTC

<210> 10

<212> 25

<212> Nucleic acid

<213> Artificial Sequence

<400> 10

GTCGACAACA TATTAATATA TAGTG

<210> 11

<212> 25

<212> Nucleic acid

<213> Artificial Sequence

<400> 11

GTCGACGTGC TCTACTTGAT TTTGC

Annex to the application documents - subsequently filed sequences listing

SEQUENCE LISTING

<110> NARA INSTITUTE OF SCIENCE AND TECHNOLOGY

<120> A METHOD FOR PROMOTING FATTY ACID SYNTHESIS IN A PLANT

<130> K54499_8

<140> 02005639.6
<141> 2002-03-12

<150> JP 2001-70691
<151> 2001-03-13

<150> JP 2001-300038
<151> 2001-09-28

<160> 11

<170> PatentIn Ver. 2.1

<210> 1
<211> 1830
<212> DNA
<213> N.tabacum cv.Xanthi

<400> 1
```
cgaatttgac acgacatagg agaagccgcc ctttattaaa aattatatta ttttaaataa 60
tataaagggg gttccaacat attaatatat agtgaagtgt tcccccagat tcagaacttt 120
ttttcaatac tcacaatcct tattagttaa taatcctagt gattggattt ctatgcttag 180
tctgatagga aataagatat tcaaataaat aattttatag cgaatgacta ttcatctatt 240
gtattttcat gcaaataggg ggcaagaaaa ctctatggaa agatggtggt ttaattcgat 300
gttgtttaag aaggagttcg aacgcaggtg tgggctaaat aaatcaatgg gcagtcttgg 360
tcctattgaa aataccaatg aagatccaaa tcgaaaagtg aaaaacattc atagttggag 420
gaatcgtgac aattctagtt gcagtaatgt tgattattta ttcggcgtta aagacattcg 480
gaatttcatc tctgatgaca ctttttagt tagtgatagg aatggagaca gttattccat 540
ctattttgat attgaaaatc atatttttga gattgacaac gatcattctt ttctgagtga 600
actagaaagt tctttttata gttatcgaaa ctcgaattat cggaataatg gatttagggg 660
cgaagatccc tactataatt cttacatgta tgatactcaa tatagttgga ataatcacat 720
taatagttgc attgatagtt atcttcagtc tcaaatctgt atagatactt ccattataag 780
tggtagtgag aattacggtg acagttacat ttatagggcc gtttgtggtg gtgaaagtcg 840
aaatagtagt gaaaacgagg gttccagtag acgaactcgc acgaagggca gtgatttaac 900
tataagagaa agttctaatg atctcgaggt aactcaaaaa tacaggcatt tgtgggttca 960
atgcgaaaat tgttatggat taaattataa gaaatttttg aaatcaaaaa tgaatatttg 1020
tgaacaatgt ggatatcatt tgaaaatgag tagttcagat agaattgaac ttttgatcga 1080
tccgggtact tgggatccta tggatgaaga catggtctct ctagatccca ttgaatttca 1140
ttcggaggag gagccttata aagatcgtat tgattcttat caaagaaaga caggattaac 1200
cgaggctgtt caaacaggca taggccaact aaacggcatt cccgtagcaa ttggggttat 1260
```

```
ggattttcag tttatggggg gtagtatggg atccgtagtc ggagagaaaa tcacccgttt 1320
gattgaatac gctgccaatc aaattttacc ccttattata gtgtgtgctt ctgggggggc 1380
gcgcatgcag gaaggaagtt tgagcttgat gcaaatggct aaaatatcgt ctgctttata 1440
tgattatcaa ttaaataaaa agttatttta tgtatcaatc cttacatctc cgacaactgg 1500
tggagtgaca gctagttttg gtatgttggg ggatatcatt attgccgaac ccaacgccta 1560
cattgcattt gcaggtaaaa gagtaattga acaaacattg aataaaacag tacccgaagg 1620
ttcacaagca gctgaatact tattccagaa gggtttattc gacctaattg taccacgtaa 1680
tcttttaaaa agcgttctga gtgagttatt taagctccac gcctttttc ctttgaatca 1740
aaagtcaagc aaaatcaagt agagcactaa gttcaattat tttatttgtg tttgtagcaa 1800
aaaagtagtt agtttgtcgg aatcaaagta                                  1830
```

<210> 2
<211> 2150
<212> DNA
<213> N.tabacum cv.Xanthi

<400> 2

```
gtcgagtaga ccttgttgtt gtgagaattc ttaattcatg agttgtaggg agggatttat 60
gtcaccacaa acagagacta aagcaagtgt tggattcaaa gctggtgtta aagagtacaa 120
attgacttat tatactcctg agtaccaaac caaggatact gatatattgg cagcattccg 180
agtaactcct caacctggag ttccacctga agaagcaggg gccgcggtag ctgccgaatc 240
ttctactggt acatggacaa ctgtatggac cgatggactt accagccttg atcgttacaa 300
agggcgatgc taccgcatcg agcgtgttgt tggagaaaaa gatcaatata ttgcttatgt 360
agcttaccct ttagaccttt ttgaagaagg ttctgttacc aacatgttta cttccattgt 420
aggtaacgta tttgggttca aagccctgcg cgctctacgt ctggaagatc tgcgaatccc 480
tcctgcttat gttaaaactt tccaaggtcc gcctcatggg atccaagttg aaagagataa 540
attgaacaag tatggtcgtc ccctgttggg atgtactatt aaacctaaat tggggttatc 600
tgctaaaaac tacggtagag ctgtttatga atgtcttcgc ggtggacttg attttaccaa 660
agatgatgag aacgtgaact cacaaccatt tatgcgttgg agagatcgtt tcttattttg 720
tgccgaagca ctttataaag cacaggctga aacaggtgaa atcaaagggc attacttgaa 780
tgctactgca ggtacatgcg aagaaatgat caaaagagct gtatttgcta gagaattggg 840
cgttccgatc gtaatgcatg actacttaac ggggggattc accgcaaata ctagcttggc 900
tcattattgc cgagataatg gtctacttct tcacatccac cgtgcaatgc atgcggttat 960
tgatagacag aagaatcatg gtatccactt ccgggtatta gcaaaagcgt tacgtatgtc 1020
tggtggagat catattcact ctggtaccgt agtaggtaaa cttgaaggtg aaagagacat 1080
aactttgggc tttgttgatt tactgcgtga tgattttgtt gaacaagatc gaagtcgcgg 1140
tatttatttc actcaagatt gggtctcttt accaggtgtt ctacccgtgg cttcaggagg 1200
tattcacgtt tggcatatgc ctgctctgac cgagatcttt ggggatgatt ccgtactaca 1260
gttcggtgga ggaactttag gacatccttg ggtaatgcg ccaggtgccg tagctaatcg 1320
agtagctcta gaagcatgtg taaaagctcg taatgaagga cgtgatcttg ctcaggaagg 1380
taatgaaatt attcgcgagg cttgcaaatg gagcccggaa ctagctgctg cttgtgaagt 1440
atggaaagag atcgtattta attttgcagc agtggacgtt ttggataagt aaaaacagta 1500
gacattagca gataaattag caggaaataa agaaggataa ggagaaagaa ctcaagtaat 1560
tatccttcgt tctcttaatt gaattgcaat taaactcggc ccaatctttt actaaaagga 1620
ttgagccgaa tacaacaaag attctattgc atatattttg actaagtata tacttaccta 1680
gatatacaag atttgaaata caaaatctag aaaactaaat caaaatctaa gactcaaatc 1740
tttctattgt tgtcttggat ccacaattaa tcctacggat ccttaggatt ggtatattct 1800
```

tttctatcct gtagtttgta gtttccctga atcaagccaa gtatcacacc tctttctacc 1860
catcctgtat attgtcccct ttgttccgtg ttgaaataga accttaattt attacttatt 1920
tttttattaa attttagatt tgttagtgat tagatattag tattagacga gattttacga 1980
aacaattatt tttttatttc tttataggag aggacaaatc tctttttcg atgcgaattt 2040
gacacgacat aggagaagcc gcccttatt aaaaattata ttattttaaa taatataaag 2100
ggggttccaa catattaata tatagtgaag tgttcccca gattcagaac 2150

<210> 3
<211> 220
<212> DNA
<213> N.tabacum cv.Xanthi

<400> 3
gcccaatgtg agtttttcta gttggatttg ctcccccgcc gtcgttcaat gagaatggat 60
aagaggctcg tgggattgac gtgaggggc agggatggct atatttctgg gagcgaactc 120
cgggcgaata tgaagcgcat ggatacaagt tatgccttgg aatgaaagac aattccgaat 180
ccgctttgtc tacgaacaag gaagctataa gtaatgcaac 220

<210> 4
<211> 25
<212> DNA
<213> Artificial Sequence

<220>
<223> Description of Artificial Sequence: Nucleic Acid

<400> 4
gtcgagtaga ccttgttgtt gtgag 25

<210> 5
<211> 32
<212> DNA
<213> Artificial Sequence

<220>
<223> Description of Artificial Sequence: Nucleic Acid

<400> 5
cccgggcggc cgcggaaccc cctttatatt at 32

<210> 6
<211> 32
<212> DNA
<213> Artificial Sequence

<220>
<223> Description of Artificial Sequence: Nucleic Acid

<400> 6
ccgcggccgc ccggggtctg ataggaaata ag 32

<210> 7
<211> 25
<212> DNA
<213> Artificial Sequence

<220>
<223> Description of Artificial Sequence: Nucleic Acid

<400> 7
gtcgacgtgc tctacttgat tttgc 25

<210> 8
<211> 24
<212> DNA
<213> Artificial Sequence

<220>
<223> Description of Artificial Sequence: Nucleic Acid

<400> 8
gtcgacgctc ccccgccgtc gttc 24

<210> 9
<211> 27
<212> DNA
<213> Artificial Sequence

<220>
<223> Description of Artificial Sequence: Nucleic Acid

<400> 9
ggtacccggg attcggaatt gtctttc 27

<210> 10
<211> 25
<212> DNA
<213> Artificial Sequence

```
<220>
<223> Description of Artificial Sequence: Nucleic Acid

<400> 10
gtcgacaaca tattaatata tagtg                                        25


<210> 11
<211> 25
<212> DNA
<213> Artificial Sequence

<220>
<223> Description of Artificial Sequence: Nucleic Acid

<400> 11
gtcgacgtgc tctacttgat tttgc                                        25
```

## Claims

**1.** A method for promoting fatty acid synthesis in a plant, the method comprising over-expression of the *accD* gene of plastidic acetyl-CoA carboxylase.

**2.** The method according to Claim 1, the method comprising the steps of preparing a construct, the construct comprising a plasmid harboring (1) *accD* gene, (2) a promoter sequence of a gene exhibiting abundant expression in plastids and chloroplasts, and (3) a homologous sequence that enables homologous recombination in plastid genome and conducting plastid transformation of a plant by the construct to over-express said *accD* gene.

**3.** The method according to Claim 2, wherein said homologous sequence that enables homologous recombination in plastid genome is a sequence comprising Rubisco large subunit gene described in SEQ.ID.NO:2 in the sequence listing and its downstream sequence, and said promoter sequence of a gene exhibiting abundant expression in plastids and chloroplasts is ribosomal RNA operon 16 promoter sequence described in SEQ.ID.NO:3 in the sequence listing.

**4.** The method according to any one of Claims 1 to 3, wherein over-expression of the *accD* gene causes increased carboxytransferase (CT) beta subunit accompanied with increased other subunits constituting plastidic acetyl-CoA carboxylase.

**5.** The method according to Claim 4, wherein said other subunits constituting plastidic acetyl-CoA carboxylase are biotin carboxylase (BC) protein, biotin carboxyl-carrier protein (BCCP) and carboxytransferase (CT) alpha subunit protein.

**6.** A transformed plant wherein fatty acid synthesis is promoted in the plant according to the method according to any one of Claims 1 to 5.

**7.** A transformed plant wherein chloroplasts of said plant are transformd by a construct comprising a plasmid harboring (1) *accD* gene, (2) a promoter sequence of a gene exhibiting abundant expression in plastids and chloroplasts, and (3) a homologous sequence that enables homologous recombination in plastid genome.

8. The transformed plant according to Claim 7, wherein said homologous sequence that enables homologous recombination in plastid genome is a sequence comprising Rubisco large subunit gene described in SEQ.ID.NO:2 in the sequence listing and its 3' downstream sequence, and said promoter sequence of a gene exhibiting abundant expression in plastids and chloroplasts is ribosomal RNA operon 16 promoter sequence described in SEQ.ID.NO: 3 in the sequence listing.

9. The transformed plant according to Claim 7 or 8, wherein fatty acid content increases and the starch content decreases in leaves of said plant as compared with a plant of control line.

10. The transformed plant according to any one of Claims 7 to 9, wherein the leaf longevity of the plant extended as compared with that of a plant of control line.

11. The transformed plant according to any one of Claims 7 to 10, wherein the fatty acid content increases and the starch content decreases in seeds of said plant as compared with a plant of control line.

12. The transformed plant according to any one of Claims 7 to 11, wherein the number of seeds per a plant body increases as compared with a plant of control line, thereby the amount of total fatty acids per the plant body increases as compared with a plant of control line.

13. The transformed plant according to any one of Claims 7 to 12, wherein the ratio of fatty acids to starch is improved as compared with a plant of control line.

14. The transformed plant according to any one of Claims 7 to 13, wherein said plant is tobacco.

15. A seed obtained from the transformed plant according to any one of Claims 6 to 14.

16. A construct comprising a plasmid harboring (1) *accD* gene, (2) a promoter sequence of a gene exhibiting abundant expression in plastids and chloroplasts, and (3) a homologous sequence that enables homologous recombination in plastid genome.

17. A method for promoting fatty acid synthesis in a plant, the method comprising over-expression of the *accD* operon containing a gene (*accD*) of plastidic acetyl-CoA carboxylase, *psaI, ycf4, cemA,* and *petA*.

FIG. 1

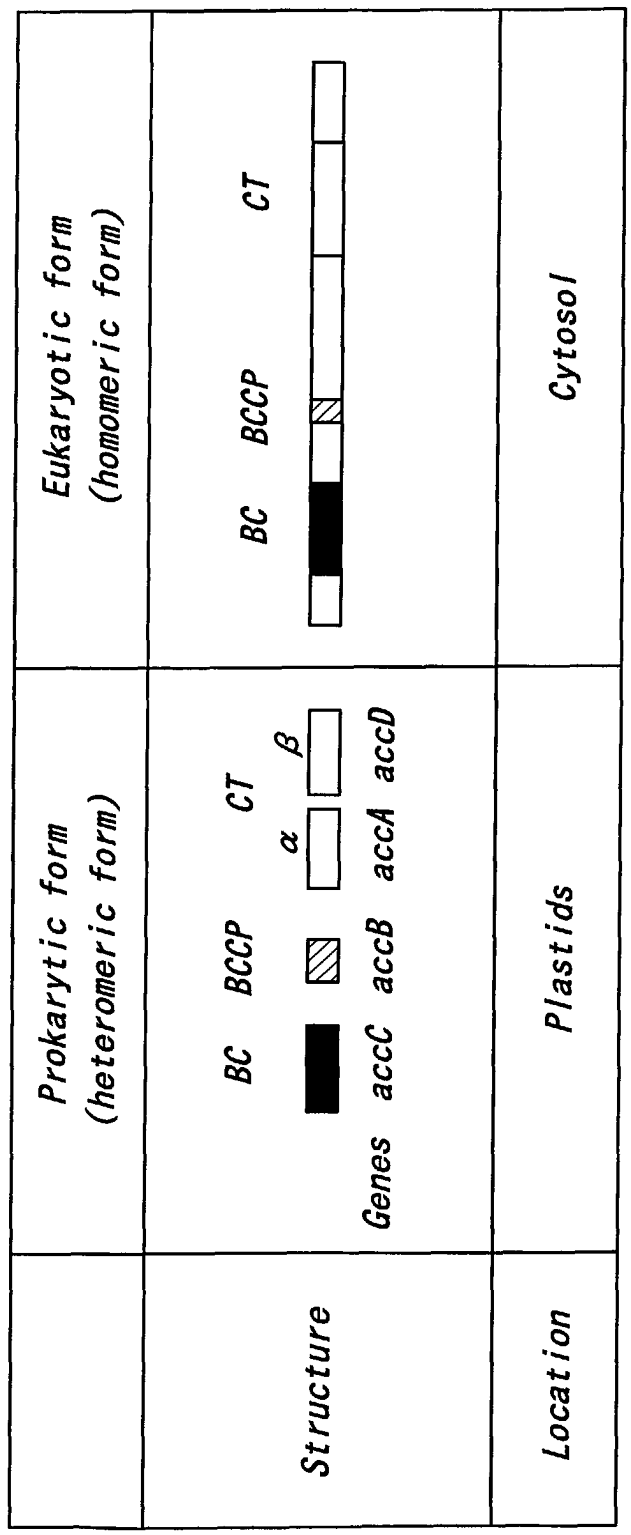
Prokaryotic form
(heteromeric form)
Eukaryotic form
(homomeric form)
Structure
BC
BCCP
CT
α
β
Genes
accC
accB
accA
accD
BC
BCCP
CT
Location
Plastids
Cytosol

# FIG. 2

↓P

1 CGAATTTGACACGACATAGGAGAAGCCGCCCTTTATTAAAAATTATATTATTTTAAATAA 60
61 TATAAAGGGGGTTCCAACATATTAATATATAGTGAAGTGTTCCCCCAGATTCAGAACTTT 120
121 TTTTCAATACTCACAATCCTTATTAGTTAATAATCCTAGTGATTGGATTTCTATGCTTAG 180
181 TCTGATAGGAAATAAGATATTCAAATAAATAATTTTATAGCGAATGACTATTCATCTATT 240
241 GTATTTTCATGCAAATAGGGGGCAAGAAAACTCTATGGAAAGATGGTGGTTTAATTCGAT 300
301 GTTGTTTAAGAAGGAGTTCGAACGCAGGTGTGGGCTAAATAAATCAATGGGCAGTCTTGG 360
361 TCCTATTGAAAATACCAATGAAGATCCAAATCGAAAAGTGAAAAACATTCATAGTTGGAG 420
421 GAATCGTGACAATTCTAGTTGCAGTAATGTTGATTATTTATTCGGCGTTAAAGACATTCG 480
481 GAATTTCATCTCTGATGACACTTTTTTAGTTAGTGATAGGAATGGAGACAGTTATTCCAT 540
541 CTATTTTGATATTGAAAATCATATTTTTGAGATTGACAACGATCATTCTTTTCTGAGTGA 600
601 ACTAGAAAGTTCTTTTTATAGTTATCGAAACTCGAATTATCGGAATAATGGATTTAGGGG 660
661 CGAAGATCCCTACTATAATTCTTACATGTATGATACTCAATATAGTTGGAATAATCACAT 720
721 TAATAGTTGCATTGATAGTTATCTTCAGTCTCAAATCTGTATAGATACTTCCATTATAAG 780
781 TGGTAGTGAGAATTACGGTGACAGTTACATTTATAGGGCCGTTTGTGGTGGTGAAAGTCG 840
841 AAATAGTAGTGAAAACGAGGGTTCCAGTAGACGAACTCGCACGAAGGGCAGTGATTTAAC 900
901 TATAAGAGAAAGTTCTAATGATCTCGAGGTAACTCAAAAATACAGGCATTTGTGGGTTCA 960
961 ATGCGAAAATTGTTATGGATTAAATTATAAGAAATTTTTGAAATCAAAAATGAATATTTG 1020
1021 TGAACAATGTGGATATCATTTGAAAATGAGTAGTTCAGATAGAATTGAACTTTTGATCGA 1080
1081 TCCGGGTACTTGGGATCCTATGGATGAAGACATGGTCTCTCTAGATCCCATTGAATTTCA 1140
1141 TTCGGAGGAGGAGCCTTATAAAGATCGTATTGATTCTTATCAAAGAAAGACAGGATTAAC 1200
1201 CGAGGCTGTTCAAACAGGCATAGGCCAACTAAACGGCATTCCCGTAGCAATTGGGGTTAT 1260
1261 GGATTTTCAGTTTATGGGGGGTAGTATGGGATCCGTAGTCGGAGAGAAAATCACCCGTTT 1320
1321 GATTGAATACGCTGCCAATCAAATTTTACCCCTTATTATAGTGTGTGCTTCTGGGGGGGC 1380
1381 GCGCATGCAGGAAGGAAGTTTGAGCTTGATGCAAATGGCTAAAATATCGTCTGCTTTATA 1440
1441 TGATTATCAATTAAATAAAAAGTTATTTTATGTATCAATCCTTACATCTCCGACAACTGG 1500
1501 TGGAGTGACAGCTAGTTTTGGTATGTTGGGGGATATCATTATTGCCGAACCCAACGCCTA 1560
1561 CATTGCATTTGCAGGTAAAAGAGTAATTGAACAAACATTGAATAAAACAGTACCCGAAGG 1620
1621 TTCACAAGCAGCTGAATACTTATTCCAGAAGGGTTTATTCGACCTAATTGTACCACGTAA 1680
1681 TCTTTTAAAAAGCGTTCTGAGTGAGTTATTTAAGCTCCACGCCTTTTTTCCTTTGAATCA 1740
1741 AAAGTCAAGCAAAATCAAGTAGAGCACTAAGTTCAATTATTTTATTTGTGTTTGTAGCAA 1800
1801 AAAAGTAGTTAGTTTGTCGGAATCAAAGTA 1830

—— : *Promoter sequence of accD gene*
═══ : *Translation initiation codon of accD gene*
- - : *Translation termination codon of accD gene*
P: *Transcription initiation site of accD gene*

# FIG. 3

| | | |
|---|---|---|
| 1 | GTCGAGTAGACCTTGTTGTTGTGAGAATTCTTAATTCATGAGTTGTAGGGAGGGATTTAT | 60 |
| 61 | GTCACCACAAACAGAGACTAAAGCAAGTGTTGGATTCAAAGCTGGTGTTAAAGAGTACAA | 120 |
| 121 | ATTGACTTATTATACTCCTGAGTACCAAACCAAGGATACTGATATATTGGCAGCATTCCG | 180 |
| 181 | AGTAACTCCTCAACCTGGAGTTCCACCTGAAGAAGCAGGGGCCGCGGTAGCTGCCGAATC | 240 |
| 241 | TTCTACTGGTACATGGACAACTGTATGGACCGATGGACTTACCAGCCTTGATCGTTACAA | 300 |
| 301 | AGGGCGATGCTACCGCATCGAGCGTGTTGTTGGAGAAAAAGATCAATATATTGCTTATGT | 360 |
| 361 | AGCTTACCCTTTAGACCTTTTTGAAGAAGGTTCTGTTACCAACATGTTTACTTCCATTGT | 420 |
| 421 | AGGTAACGTATTTGGGTTCAAAGCCCTGCGCGCTCTACGTCTGGAAGATCTGCGAATCCC | 480 |
| 481 | TCCTGCTTATGTTAAAACTTTCCAAGGTCCGCCTCATGGGATCCAAGTTGAAAGAGATAA | 540 |
| 541 | ATTGAACAAGTATGGTCGTCCCCTGTTGGGATGTACTATTAAACCTAAATTGGGGTTATC | 600 |
| 601 | TGCTAAAAACTACGGTAGAGCTGTTTATGAATGTCTTCGCGGTGGACTTGATTTTACCAA | 660 |
| 661 | AGATGATGAGAACGTGAACTCACAACCATTTATGCGTTGGAGAGATCGTTTCTTATTTTG | 720 |
| 721 | TGCCGAAGCACTTTATAAAGCACAGGCTGAAACAGGTGAAATCAAAGGGCATTACTTGAA | 780 |
| 781 | TGCTACTGCAGGTACATGCGAAGAAATGATCAAAAGAGCTGTATTTGCTAGAGAATTGGG | 840 |
| 841 | CGTTCCGATCGTAATGCATGACTACTTAACGGGGGGATTCACCGCAAATACTAGCTTGGC | 900 |
| 901 | TCATTATTGCCGAGATAATGGTCTACTTCTTCACATCCACCGTGCAATGCATGCGGTTAT | 960 |
| 961 | TGATAGACAGAAGAATCATGGTATCCACTTCCGGGTATTAGCAAAAGCGTTACGTATGTC | 1020 |
| 1021 | TGGTGGAGATCATATTCACTCTGGTACCGTAGTAGGTAAACTTGAAGGTGAAAGAGACAT | 1080 |
| 1081 | AACTTTGGGCTTTGTTGATTTACTGCGTGATGATTTTGTTGAACAAGATCGAAGTCGCGG | 1140 |
| 1141 | TATTTATTTCACTCAAGATTGGGTCTCTTTACCAGGTGTTCTACCCGTGGCTTCAGGAGG | 1200 |
| 1201 | TATTCACGTTTGGCATATGCCTGCTCTGACCGAGATCTTTGGGGATGATTCCGTACTACA | 1260 |
| 1261 | GTTCGGTGGAGGAACTTTAGGACATCCTTGGGGTAATGCGCCAGGTGCCGTAGCTAATCG | 1320 |
| 1321 | AGTAGCTCTAGAAGCATGTGTAAAAGCTCGTAATGAAGGACGTGATCTTGCTCAGGAAGG | 1380 |
| 1381 | TAATGAAATTATTCGCGAGGCTTGCAAATGGAGCCCGGAACTAGCTGCTGCTTGTGAAGT | 1440 |
| 1441 | ATGGAAAGAGATCGTATTTAATTTTGCAGCAGTGGACGTTTTGGATAAGTAAAAACAGTA | 1500 |
| 1501 | GACATTAGCAGATAAATTAGCAGGAAATAAAGAAGGATAAGGAGAAAGAACTCAAGTAAT | 1560 |
| 1561 | TATCCTTCGTTCTCTTAATTGAATTGCAATTAAACTCGGCCCAATCTTTTACTAAAAGGA | 1620 |
| 1621 | TTGAGCCGAATACAACAAAGATTCTATTGCATATATTTTGACTAAGTATATACTTACCTA | 1680 |
| 1681 | GATATACAAGATTTGAAATACAAAATCTAGAAAACTAAATCAAAATCTAAGACTCAAATC | 1740 |
| 1741 | TTTCTATTGTTGTCTTGGATCCACAATTAATCCTACGGATCCTTAGGATTGGTATATTCT | 1800 |
| 1801 | TTTCTATCCTGTAGTTTGTAGTTTCCCTGAATCAAGCCAAGTATCACACCTCTTTCTACC | 1860 |
| 1861 | CATCCTGTATATTGTCCCCTTTGTTCCGTGTTGAAATAGAACCTTAATTTATTACTTATT | 1920 |
| 1921 | TTTTTATTAAATTTTAGATTTGTTAGTGATTAGATATTAGTATTAGACGAGATTTTACGA | 1980 |
| 1981 | AACAATTATTTTTTTATTTCTTTATAGGAGAGGACAAATCTCTTTTTTCGATGCGAATTT | 2040 |
| 2041 | GACACGACATAGGAGAAGCCGCCCTTTATTAAAAATTATATTATTTTAAATAATATAAAG | 2100 |
| 2101 | GGGGTTCCAACATATTAATATATAGTGAAGTGTTCCCCCAGATTCAGAAC | 2150 |

═ : *Translation initiation codon of rbcL gene*

— - : *Translation termination codon of rbcL gene*

□ : *Nucleotides different from that reported by Shinozaki et al*

# FIG. 4

GCCCAATGTGAGTTTTTCTAGTTGGATTTGCTCCCCCGCCGTCGTTC

AATGAGAATGGATAAGAGGCTCGTGGGATTGACGTGAGGGGGCAGGG

P1

ATGGCTATATTTCTGGGAGCGAACTCCGGGCGAATATGAAGCGCATG

P2

GATACAAGTTATGCCTTGGAATGAAAGACAATTCCGAATCCGCTTTG

TCTACGAACAAGGAAGCTATAAGTAATGCAAC

——— *Ribosomal RNA operon (rrn)16 promoter recognized by PEP*

P1 : *Transcription initiation site of PEP promoter*

- - - - - *rrn16 promoter recognized by NEP*

P2 : *Transcription initiation site of NEP promoter*

FIG. 5

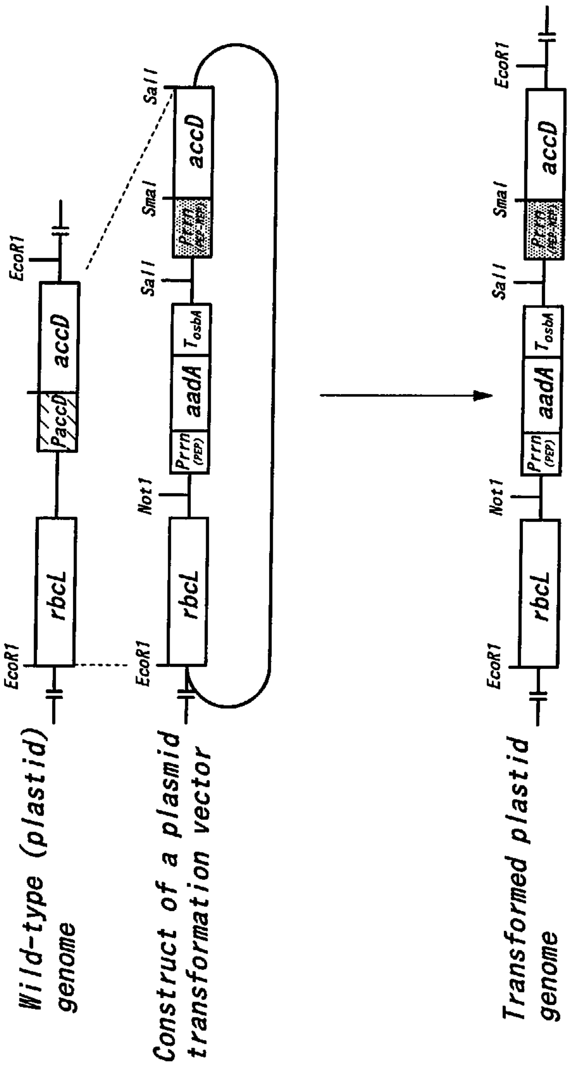
Wild-type (plastid) genome
EcoR1
rbcL
PaccD
accD
EcoR1
Construct of a plasmid transformation vector
EcoR1
rbcL
Not1
Prrn (PEP)
aadA
TpsbA
Sal1
Prrn (PEP+NEP)
Sma1
accD
Sal1
Transformed plastid genome
EcoR1
rbcL
Not1
Prrn (PEP)
aadA
TpsbA
Sal1
Prrn (PEP+NEP)
Sma1
accD
EcoR1

FIG. 6

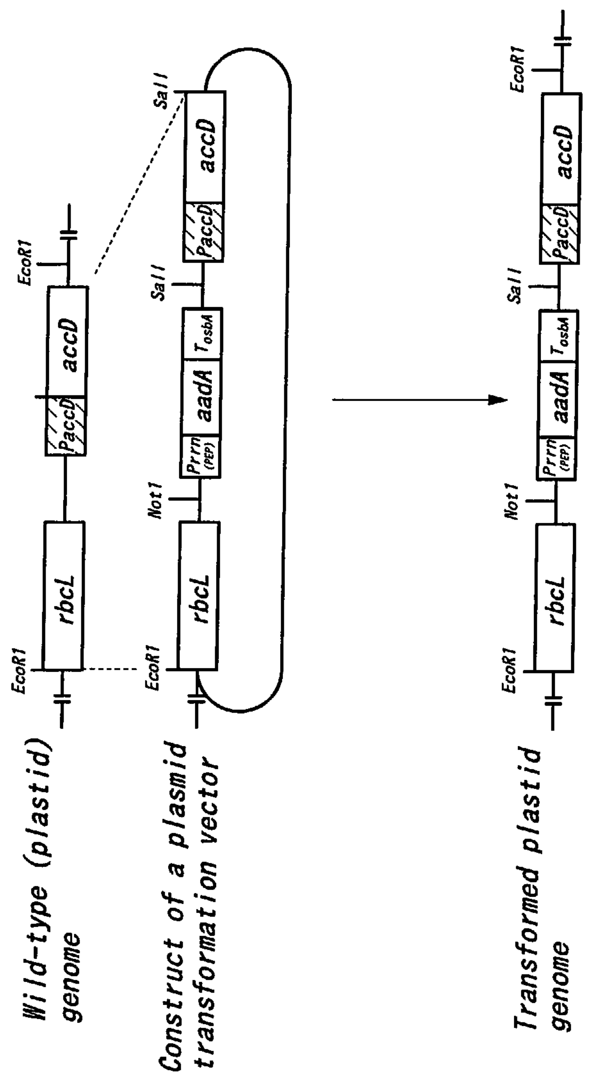
Wild-type (plastid) genome
Construct of a plasmid transformation vector
Transformed plastid genome
EcoR1
rbcL
PaccD
accD
Not1
Prrn (PEP)
aadA
TpsbA
Sal1

FIG. 7

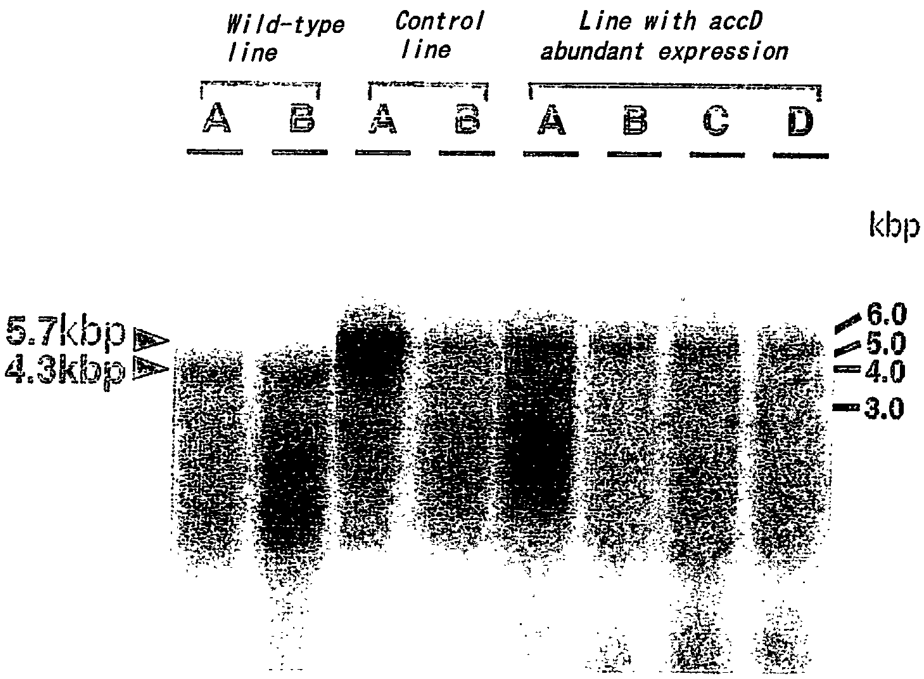
Wild-type line
Control line
Line with accD abundant expression
A B A B A B C D
kbp
5.7kbp
4.3kbp
6.0
5.0
4.0
3.0

FIG. 8

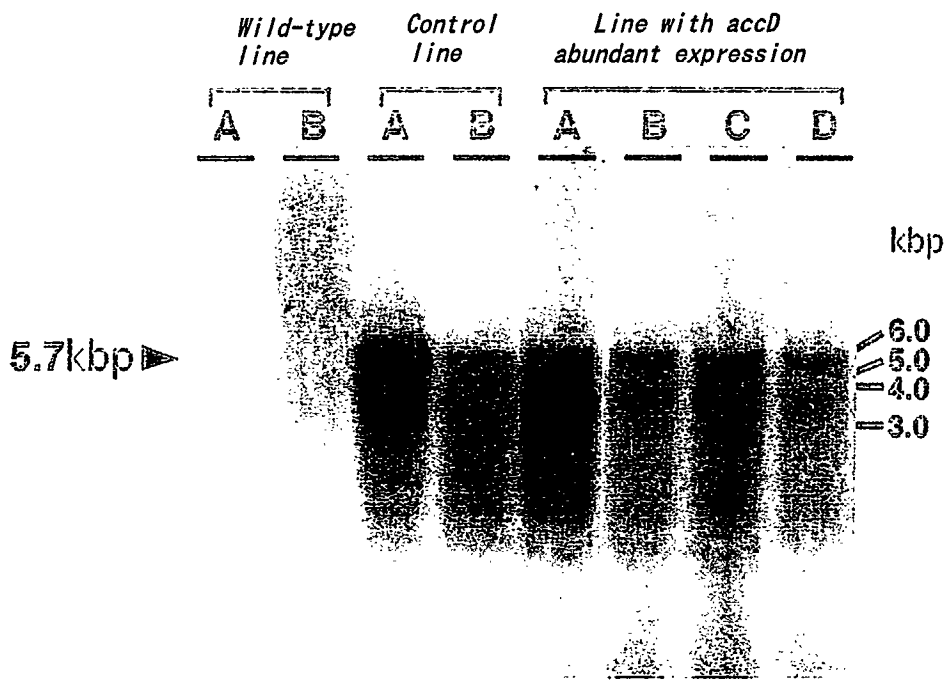
Wild-type line
Control line
Line with accD abundant expression
A
B
A
B
A
B
C
D
kbp
5.7kbp
6.0
5.0
4.0
3.0

## FIG. 9

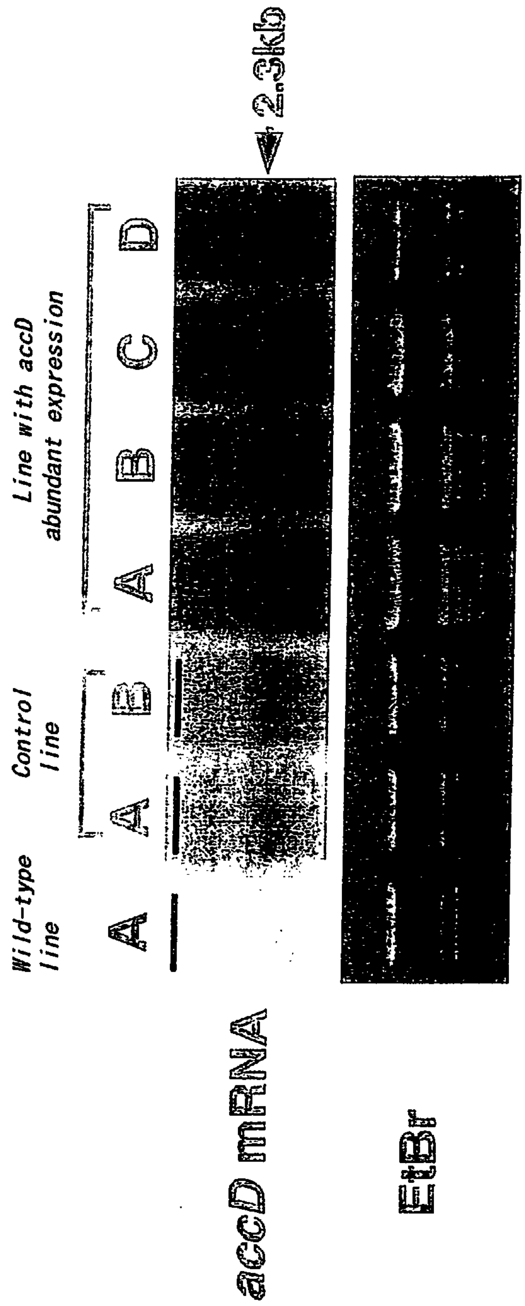
Wild-type line
Control line
Line with accD abundant expression
A
A
B
A
B
C
D
2.3kb
accD mRNA
EtBr

FIG. 10

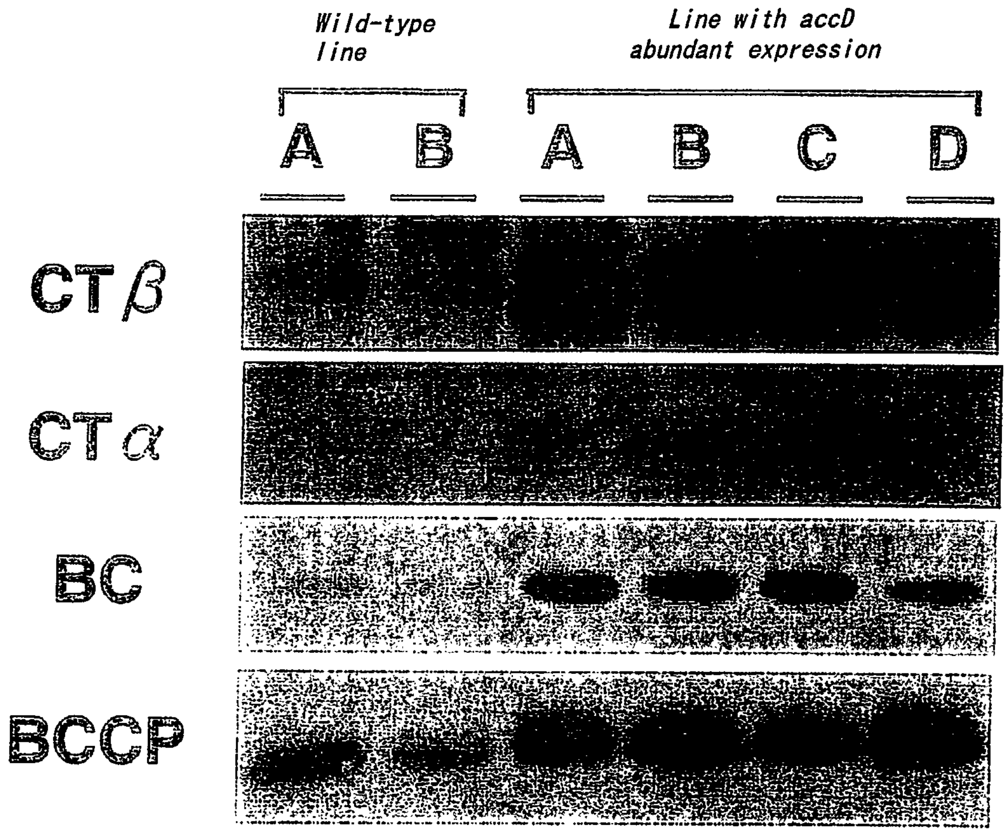
Wild-type line
Line with accD abundant expression
A
B
A
B
C
D
CTβ
CTα
BC
BCCP

FIG. 11

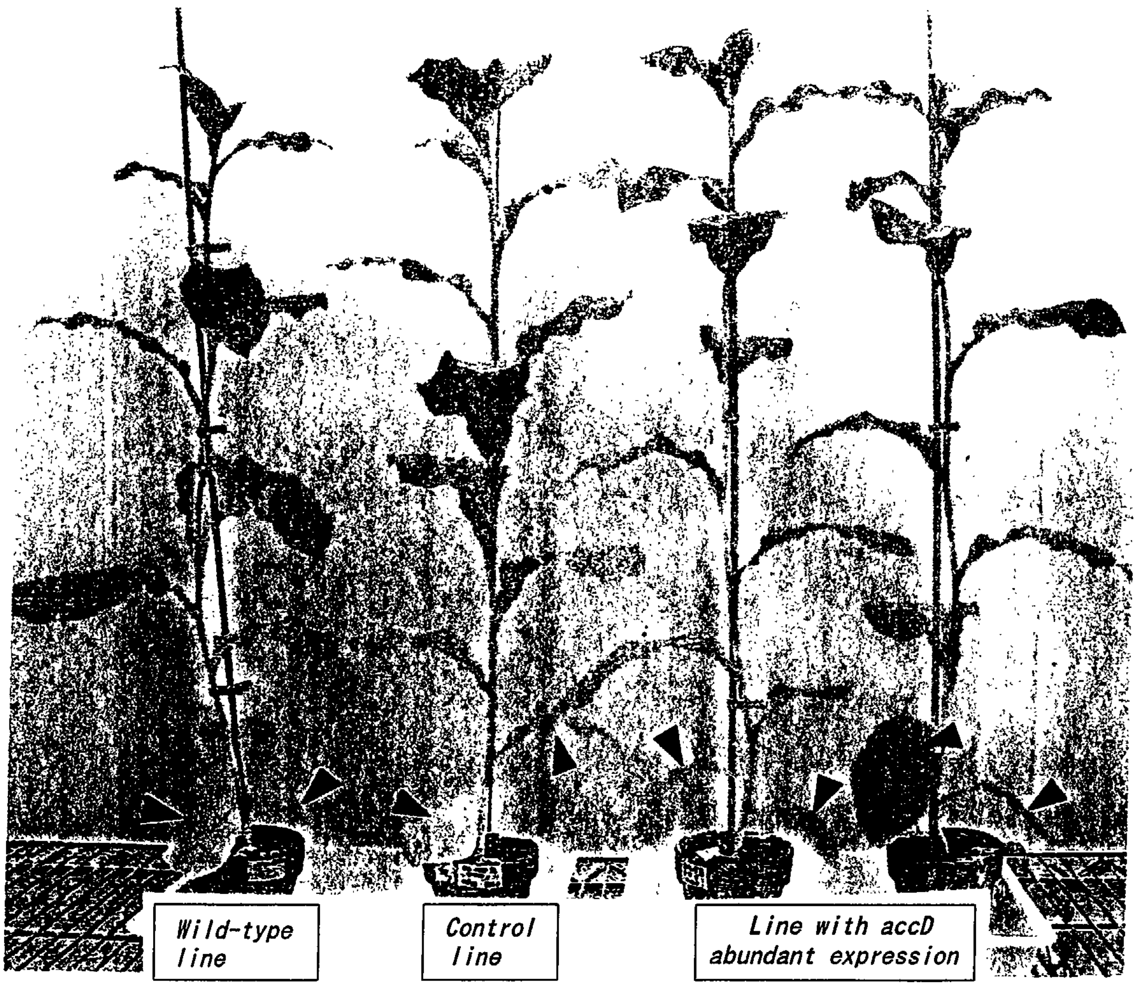
Wild-type line
Control line
Line with accD abundant expression

# FIG. 12

Wild-type line

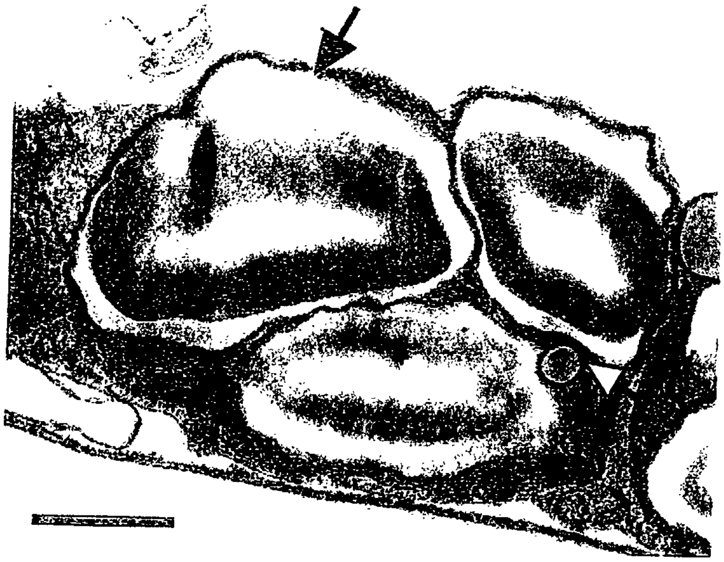

# FIG. 13

Line with accD abundant expression

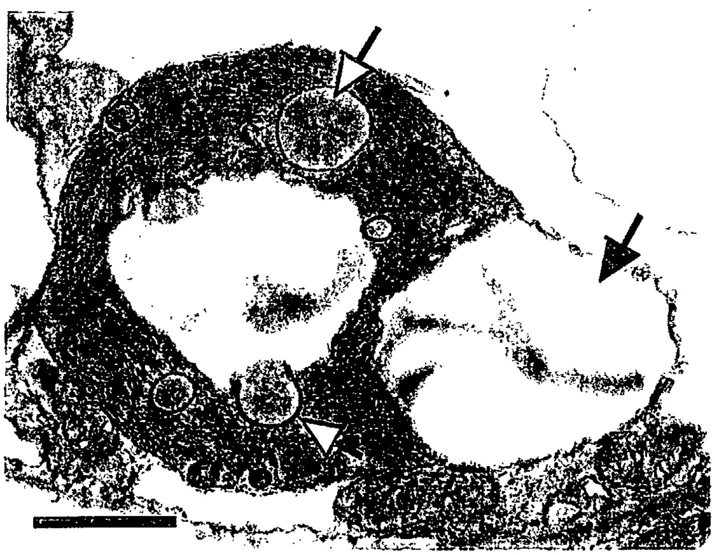

FIG. 14

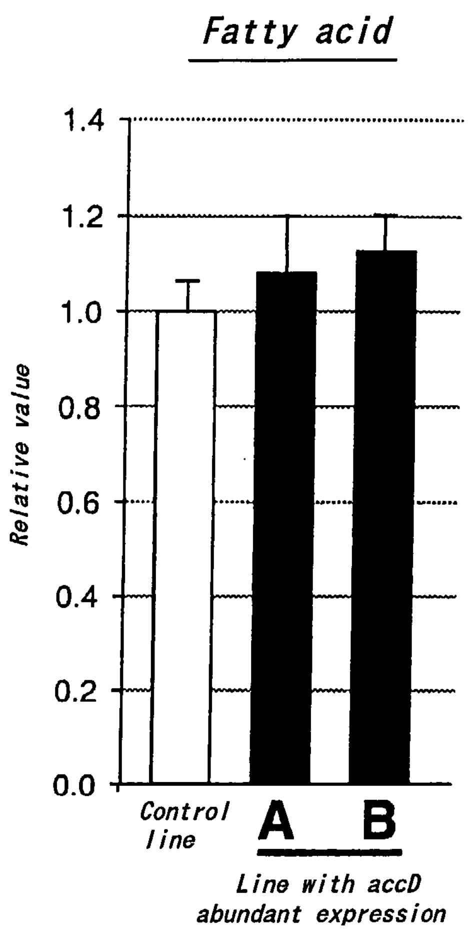
Fatty acid
Relative value
1.4
1.2
1.0
0.8
0.6
0.4
0.2
0.0
Control line
A
B
Line with accD abundant expression

FIG. 15

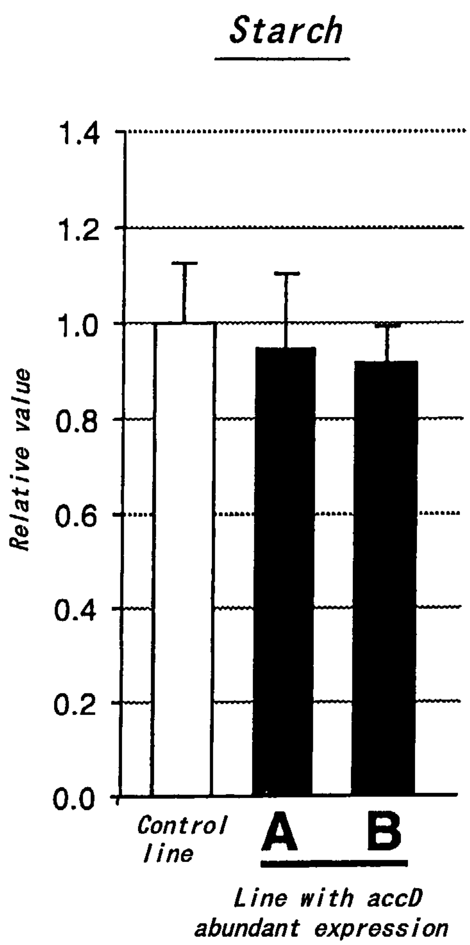
Starch
Relative value
1.4
1.2
1.0
0.8
0.6
0.4
0.2
0.0
Control line
A
B
Line with accD abundant expression

FIG. 16

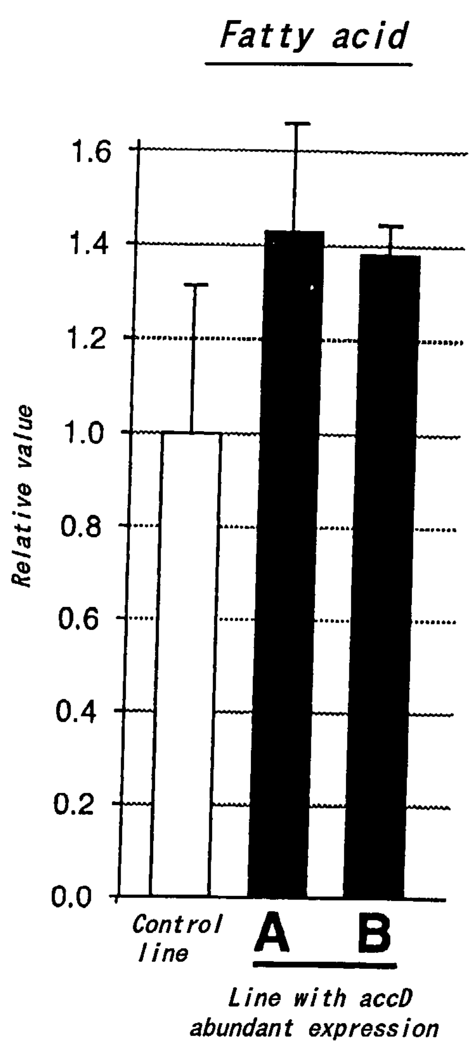
Fatty acid
Relative value
1.6
1.4
1.2
1.0
0.8
0.6
0.4
0.2
0.0
Control line
A
B
Line with accD abundant expression

FIG. 17

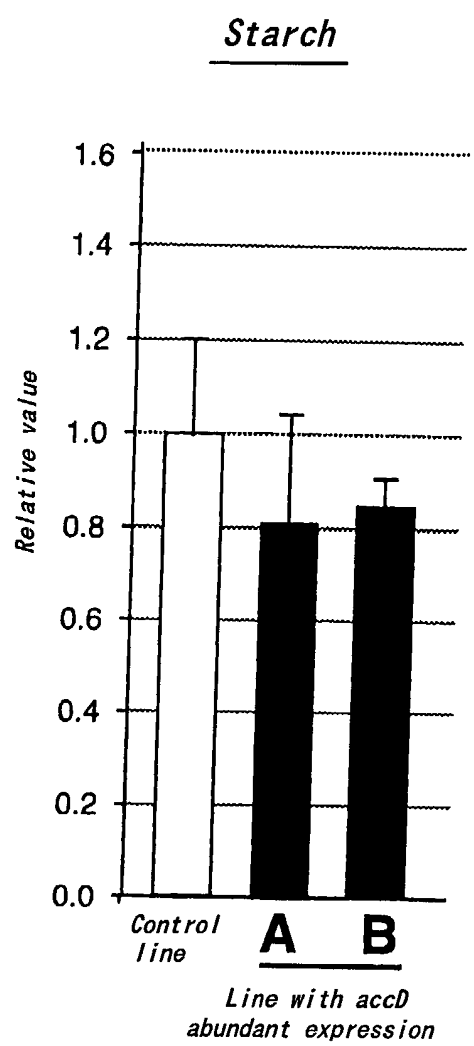
Starch
Relative value
1.6
1.4
1.2
1.0
0.8
0.6
0.4
0.2
0.0
Control line
A
B
Line with accD abundant expression

FIG. 18

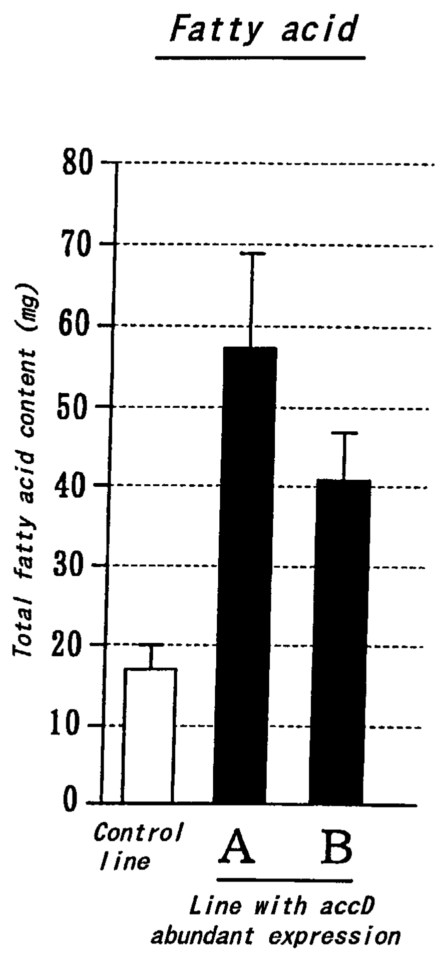
Fatty acid
Total fatty acid content (mg)
80
70
60
50
40
30
20
10
0
Control line
A
B
Line with accD abundant expression

FIG. 19

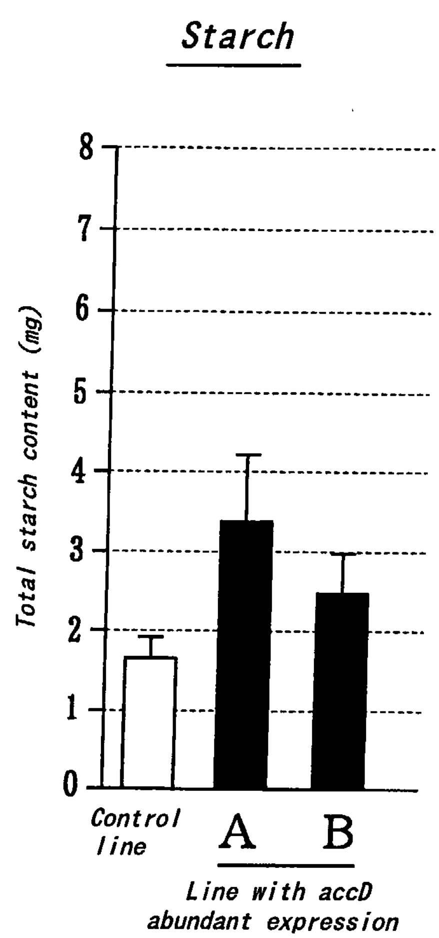
Starch
Total starch content (mg)
8
7
6
5
4
3
2
1
0
Control line
A
B
Line with accD abundant expression

FIG. 20

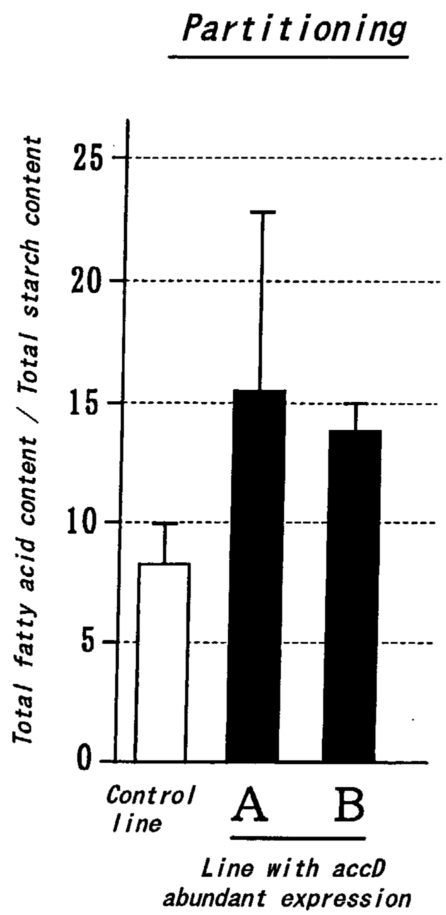
Partitioning
Total fatty acid content / Total starch content
25
20
15
10
5
0
Control line
A
B
Line with accD abundant expression